(19) Europäisches Patentamt
European Patent Office
Office européen des brevets

(11) EP 0 885 914 B1

(12) **EUROPÄISCHE PATENTSCHRIFT**

(45) Veröffentlichungstag und Bekanntmachung des Hinweises auf die Patenterteilung:
**26.02.2003 Patentblatt 2003/09**

(51) Int Cl.[7]: **C08G 65/32**, A61K 7/00, A61K 9/107, A61K 9/51

(21) Anmeldenummer: **98109953.4**

(22) Anmeldetag: **02.06.1998**

(54) **Mittels kovalent verknüpfter Netzwerke vernetzte, disperse Zwei- oder Mehr-phasensysteme**

Disperse two or multi-phase systems linked by covalent bonds into a network

Système dispersé di- ou multiphase, lié par un réseau covalent réticulé

(84) Benannte Vertragsstaaten:
**AT BE CH DE ES FR GB IT LI NL SE**

(30) Priorität: **20.06.1997 DE 19726131**

(43) Veröffentlichungstag der Anmeldung:
**23.12.1998 Patentblatt 1998/52**

(73) Patentinhaber: **Beiersdorf Aktiengesellschaft**
**20245 Hamburg (DE)**

(72) Erfinder:
- **Schreiber, Jörg, Dr.**
  **22087 Hamburg (DE)**
- **Meier, Wolfgang, Dr.**
  **4053 Basel (CH)**

(56) Entgegenhaltungen:
**WO-A-96/28132**
**WO-A-98/13025**
**WO-A-98/15255**
**DE-A- 4 431 773**

- **MEIER W. ET AL: "Microemulsion Elastomers" COLLOID AND POLYMER SCIENCE , Bd. 274, Nr. 03, 1996, Seiten 218-226, XP002085476**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 885 914 B1

Printed by Jouve, 75001 PARIS (FR)

## Beschreibung

Die vorliegende Erfindung betrifft mittels kovalent verknüpfter Netzwerke vernetzte, disperse Zwei- oder Mehrphasensysteme, insbesondere solcher Zwei- oder Mehrphasensysteme, welche landläufig als einfache oder multiple Emulsionen oder Hydrodispersionen, also im wesentlichen emulgatorfreien Zubereitungen vom Typ Öl-in-Wasser bezeichnet werden, oder auch Dispersionen von lipoiden Festkörpern in einer kontinuierlichen wäßrigen Phase.

Als besondere Ausführungsformen betrifft die vorliegende Erfindung Materialien, die als Gele eingesetzt werden können. Solche Gele können vorteilhaft fließfähig, aber auch zäh sein und sogar mehr oder weniger leicht verformbare Festkörper - beispielsweise in Form von Folien oder Stiften - darstellen.

Anwendungsgebiete für den Gegenstand der vorliegenden Erfindung sind mannigfaltig. So betrifft die vorliegende Erfindung vorteilhaft kosmetische oder dermatologische Gele auf der Basis von Emulsionen, bevorzugt Mikroemulsionen, insbesondere von Mikroemulsionen vom Typ Öl-in-Wasser. Femer betrifft die vorliegende Erfindung Verfahren zur Herstellung sowie ihre Verwendung für kosmetische, medizinische und technische Zwecke bzw. für den Allgemeingebrauch.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

Hydrodispersionsgele können als Materialien ausgestaltet sein, die aufgrund eines hohen Gehaltes an hydrophilen Gruppen in der Lage sind, Wasser absorptiv zu binden. Die Wasseraufnahmekapazität vieler Hydrogele beträgt das Mehrfache des Eigengewichtes der wasserfreien Substanz.

Hydrogele werden in vielfältiger Form in der Medizin eingesetzt. Besonders geeignet sind sie in der Wundversorgung; sie können Wunden vor der Austrocknung schützen, Wundsekret aufsaugen, als Matrix für Wirkstoffe aller Art dienen sowie als Basis für die Besiedelung mit autologen oder heterologen Hautzellen dienen

Die Hydrogele des Standes der Technik haben jedoch verschiedene Nachteile: Aufgrund ihrer Hydrophilie sind die meisten in Frage kommenden Stoffe wasserlöslich. Dies ist meist unerwünscht, weil derartige Produkte nicht formstabil sind. Außerdem lösen sich derartige Produkte in unerwünschter Weise am Einsatzort auf und stehen dann für den vorgesehenen Zweck nicht mehr zur Verfügung.

Andere Produkte zeichnen sich durch sehr starke Polymervernetzung aus. Dadurch werden zwar manche der Nachteile der vorgenannten Stoffklasse vermieden. Die Quellbarkeit dieser Stoffe ist jedoch weitgehend eingeschränkt oder verloren. Überdies sind die synthetischen Vernetzer, die hier eingesetzt werden, alle mehr oder weniger toxisch.

Auch selbstklebende Gele zur medizinischen Verwendung sind an sich bekannt. Diese lassen sich zwar im allgemeinen recht gut auf der Haut fixieren, haben aber meistens den Nachteil, daß ihre Wasseraufnahme- und -abgabekapazität stark eingeschränkt sind.

In EP-A-0 097 846 werden Wundbehandlungsmittel auf der Basis von Hydrogelen beschrieben. Dabei wird Gelatine in fester Form als Pulver, Schuppen oder Blatt in einer Zwei-Phasen Reaktion mit Vernetzern wie Formaldehyd, Glyoxal, Glutarsäuredialdehyd. Dicarbonsäurechloriden und/oder Diisocyanaten umgesetzt.

Der Vernetzer wirkt dabei auf die gequollene, nicht gelöste Gelatine ein. Dieses Verfahren und die daraus erhaltenen Produkte sind mit erheblichen Nachteilen behaftet, da die verwendeten Vemetzer erhebliche Zellschädigungen bewirken können.

Zudem ist das Verfahren nicht oder nur schwer reproduzierbar. Die Vernetzung hängt nicht nur von der Konzentration der verwendeten Vernetzer ab, sondern auch von Parametern wie Temperatur und Einwirkzeit der Reaktanden. Femer sind die effektive Oberfläche und das mittlere Molekulargewicht der handelsüblichen Gelatinearten deutlichen Schwankungen unterworfen, so daß auch die Eigenschaften des vernetzten Hydrogels schwer voraussehbar sind.

Weiterhin ist in EP-A-0 097 846 ein Verbandmaterial aus Hydrogelen auf Polyvinylalkoholbasis beschrieben. Als Vemetzer wird Formaldehyd eingesetzt, der, wie eingangs erwähnt, physiologisch bedenklich ist.

Auch Schäume aus Polyvinylalkohol oder Collagen sind bekannt und gebräuchlich. Da deren Matrixsubstanzen aber die voranstehend beschriebenen Nachteile haben, sind sie zur Wundversorgung allenfalls bedingt geeignet.

Weiterhin sind hydrophile Schäume aus Polyurethangelen bekannt. Die Schrift WO-88/01878 beschreibt selbstklebende Polyurethanschäume bzw. Polyurethanschaumgele, welche unter anderem Methacrylate enthalten können.

Polyurethangele auf der Basis einer Polyurethanmatrix und höhermolekularen Polyolen werden auch in EP-B-0 057 839 beschrieben. Selbsthaftende Flächengebilde aus Polyurethangelen sind aus EP-B-0 147 588 bekannt.

Es war daher eine Aufgabe der Erfindung, gelartige Materialien zu entwickeln, welche nicht die Nachteile des Standes der Technik haben und als Wundversorgungsmittel geeignet sind. Außerdem sollten die Schäume nach reproduzierbaren Verfahren wirtschaftlich herstellbar sein.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche, und sich gerade in neuerer Zeit immer weiter verbreitende kosmetische und dermatologische Zubereitungsformen sind Gele.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind. Meistens sind Gele durchsichtig. In der kosmetischen bzw. pharmazeutischen Galenik zeichnen sich Gele in aller Regel durch halbfeste, oft fließfähige Konsistenz aus.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca 1 $\mu m$ bis ca. 50 $\mu m$. Solche "Makroemulsionen" sind, ohne weitere färbende Zusätze, milchigweißgefärbt und opak. Feinere "Makroemulsionen", deren Tröpfchendurchmesser im Bereich von ca. $10^{-1}$ $\mu m$ bis ca. 1 $\mu m$ liegen, sind, wiederum ohne färbende Zusätze, bläulichweißgefärbt und undurchsichtig.

Mizellaren und molekularen Lösungen mit Partikeldurchmessern kleiner als ca. $10^{-2}$ $\mu m$, ist vorbehalten, klar und transparent zu erscheinen.

Der Tröpfchendurchmesser von transparenten bzw. transluzenten Mikroemulsionen dagegen liegt im Bereich von etwa $10^{-2}$ $\mu m$ bis etwa $10^{-1}$ $\mu m$. Solche Mikroemulsionen sind meist niedrigviskos. Die Viskosität vieler Mikroemulsionen vom O/W-Typ ist vergleichbar mit der des Wassers.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten. Auch hier ist der hohe Gehalt an Emulgatoren von Nachteil.

Vorteil von Mikroemulsionsgelen ist, daß in der dispersen Phase Wirkstoffe feindispers vorliegen können. Ein weiterer Vorteil ist, daß sie aufgrund ihrer niedrigen Viskosität versprühbar sein können. Werden Mikroemulsionen als Kosmetika verwendet, zeichnen sich entsprechende Produkte durch hohe kosmetische Eleganz aus.

Es ist an sich bekannt, die Tröpfchen einer niedrigviskosen, insbesondere dünnflüssigen Mikroemulsion mit vemetzenden Substanzen miteinander zu verknüpfen, um auf diese Weise das dreidimensionale Netzwerk eines Geles zu erhalten.

In Nachr. Chem. Techn. Lab. 43 (1995) Nr. 1, S. 9 ff. werden zur Vernetzung von Mikroemulsionströpfchen kettenförmige, hydrophile Moleküle beschrieben, welche an den beiden Kettenenden je einen hydrophoben Rest aufweisen. Jene hydrophoben Reste tauchen in Mikroemulsionströpfchen ein, wobei die hydrophilen Kettenbereiche in

der kontinuierlichen Wasserphase vorliegen. Im strengen Sinne ist es wohl nicht nötig, daß die hydrophoben Reste "eintauchen". Es kann im Einzelfalle auch durchaus genügen, wenn durch hydrophobe Wechselwirkung die hydrophoben Reste mit der Oberfläche der Mikroemulsionströpfchen in Kontakt treten und mehr oder weniger stark an dieser haften bleiben.

Als Vernetzer werden a.a.O. Polyoxyethylenglycole mit Oleylgruppen als hydrophobe Endgruppen angegeben.

Mikroemulsionen bestehen im allgemeinen aus individuellen Wasser und Öldomänen. In einem bestimmten Bereich des Phasendiagramms können sie aus emulgatorstabilisierten Tröpfchen bestehen, die in einer kontinuierlichen Wasserphase dispergiert sind. Ferner sind auch emulgatorfreien Mikroemulsionen bekannt, die durch Hochdruckhomogeniserung hergestellt werden. Die Eigenschaften dieser niedrigviskosen Mikroemulsionen können durch Einsatz von Polymeren, die aus wasserlöslichen und öllöslichen Bereichen bestehen, gezielt modifiziert werden, da die hydrophoben Segmente oder Blöcke des Polymers sich bevorzugt in den Öldomänen und die hydrophilen Segmente sich bevorzugt in der Wasserphase aufhalten.

In der Schrift WO96/28132 wurde gezeigt, daß diese Polymere, die zum Beispiel vom Triblockcopolymere vom Typ A-B-A oder aber hydrophob modifizierte wasserlösliche Polymere sein können, Mikroemulsionen in einen gelartigen Zustand überführen. Die Öltröpfchen werden dabein in ein Netzwerk eingebunden. Diese Netzwerke sind transienter Natur, da die hydrophoben Polymerenden in der Lage sind, aus den Öldomänen wieder hinaus zu diffundieren. Dieser Prozeß zerstört die Netzwerke allerdings nicht, da dieser Vorgang nur einen sehr geringen Teil der Polymerenden betrifft und ferner an einer anderen Stelle des Gels sich zum gleichen Zeitpunkt der umgekehrte Vorgang vollziehen kann, d.h. ein sich außerhalb der Tröpchen befindliches Polymerende diffundiert in das gleiche oder in ein neues Öltröpfchen zurück.

In Fig. 1 wird das erfindungsgemäße Prinzip verdeutlicht: Die als schraffierte Kreise dargestellten Mikroemulsionströpfchen einer O/W-Mikroemulsion werden durch die als Linien dargestellten Vernetzermoleküle miteinander verbunden, welche an beiden Enden durch Rechtecke symbolisierte hydrophobe ungesättigte Reste tragen. Ersichtlich ist, daß ein Emulsionströpfchen grundsätzlich auch mehrere hydrophobe ungesättigte Reste beherbergen kann, wodurch eine stärkere Vernetzung und Dreidimensionalität des Netzwerks gewährleistbar ist. Durch thermische oder photochemische Energie werden die ungesättigten Gruppen des Polymers direkt miteinander kovalent verknüpft. Durch Polymere, die keine ungesättigten Gruppen tragen, können ferner auch Teile des Mikroemulsionsnetzwerks physikalisch vernetzt bleiben, so daß sich die Flexibilität des Elastomers erhöht. Es ist dabei grundsetzlich unerheblich, ob die hydrophoben Reste "eintauchen" oder ob die hydrophoben Reste lediglich oberflächlich mit den Mikroemulsionströpfchen in Kontakt treten und mehr oder weniger stark an dieser haften.

Obwohl die in der Schrift WO96/28132 aufgeführten Gele äußerst vorteilhafte Eigenschaften aufweisen, verbleiben dennoch zuweilen rheologische Probleme, die es zu verbessern galt.

Erstaunlicherweise werden alle der Erfindung zugrundliegenden Aufgaben gelöst durch disperse Mehrphasensysteme,

- enthaltend lediglich pharmakologisch unbedenkliche Komponenten,
- umfassend mindestens eine kontinuierliche wäßrige Phase und
- mindestens eine disperse flüssige oder feste lipoide Phase,
  - wobei die einzelnen Partikel der dispersen lipoiden Phase bzw. der dispersen lipoiden Phasen durch ein makromolekulares Netzwerk miteinander verknüpft sind,
    - welches erhältlich ist durch Reaktion einer oder mehrerer Substanzen XAY,
      - deren molekulare Struktur wenigstens einen hydrophilen makromolekularen Bereich enthält, welcher mindestens zwei reaktive lipophile molekulare Bereiche verbrückt,
      - wobei diese reaktiven lipophilen molekularen Bereiche gleichartig oder unterschiedlich sein können und
      - wobei die Einzelmoleküle der Substanz oder der Substanzen XAY über die reaktiven lipophilen molekularen Bereichen miteinander intermolekulare Reaktionen eingehen können,
      - wobei keine ungesättigten Comonomere eingesetzt werden,
    - dergestalt, daß die reaktiven, lipophilen molekularen Bereiche ins Innere der Partikel der dispersen flüssigen oder festen lipoiden Phase eintauchen und/oder an die Oberfläche der Partikel der dispersen flüssigen oder festen lipoiden Phase andocken und sodann durch Einwirkung von Wärme und/oder Licht miteinander intermolekulare Reaktionen eingehen.

Zwar werden in Colloid Polym. Sci 274 (1996) 218 ff u.a. Mikroemulsionen vom Typ O/W beschrieben, die durch ABA-Triblockcopolymere und ungesättigte Comonomere in Gegenwart von Radikalinitiatoren (Azoisobuttersäurenitril, "AIBN") kovalent in den Öltröpfchen vernetzt werden und Mikroemulsionselastomere bilden. Diese Netzwerke sind auf diese Weise nicht mehr physikalisch vemetzt. Bei diesem Verfahren können die Comonomere mit sich selbst und den ungesättigten Polmerenden der ABA-Triblockcopolymere eine chemische Reaktion eingehen und auf diese Weise die Öltröpfchen "aushärten". Der vordergründige Vorteil, auf diese Weise Mikroemulsionselastomere herzustellen, liegt darin, daß, infolge der größeren Anzahl von reaktionsfähigen chemischen Verbindungen innnerhalb der Tröpfchen, chemische Reaktionen schneller gestartet werden.

Nachteilig ist aber, daß nicht ausgeschlossen werden kann, daß Restmonomere im Mikroemulsionselastomer verbleiben. Ferner können hier eine Vielzahl neuer Verbindungen entstehen, da Monomer-Monomer-Reaktionen, Monomer-Polymer-Reaktionen, Dimer-Polymer-Reaktionen etc. entstehen können und ferner weitere dem Fachmann geläufige radikalische Nebenreaktionen, so daß toxikologisch bedenkliche Verbindungen resultieren können. Ein weiterer Nachteil ist, daß durch dieses Verfahren auch der verwendete Radikalinitiator AIBN nicht aus dem Produkt entfernt werden kann und zum Beispiel nach der Applikation auf menschliches oder tierisches Gewebe unerwünschte Nebenreaktionen hervorrufen kann.

Nachteilig an diesen Mikroemulsionselastomeren ist weiterhin, daß sie für medizinische (zum Beispiel bei verletzter Haut, Wunden etc), kosmetische oder pharmazeutische Zwecke nicht geeignet sind, da hier als ölphase kurzkettige Kohlenwasserstoffe eingesetzt werden. Ein weiterer Nachteil ist, daß der Emulgator Laureth-5 auf Grund seiner Kettenlänge schleimhautreizend und daher nicht geeignet ist für die oben beschriebenen Einsatzgebiete. Femer sind diese Mikroemulsionen nur in begrenzten Temperaturbereichen (23°C bis 30°C) transparent, was ein Nachteil sein kann, wenn aus technischen Gründen die kovalente Vernetzung bei hoher Temperatur oder unterhalb von Raumtemperatur stattfinden soll.

Die in den Mehrphasensystemen erfindungsgemäß verwendeten Substanzen XAY, deren molekulare Struktur wenigstens einen hydrophilen makromolekularen Bereich enthält, welcher mindestens zwei reaktive lipophile molekulare Bereiche verbrückt, wobei diese reaktiven lipophilen molekularen Bereiche gleichartig oder unterschiedlich sein können und wobei die Einzelmoleküle der Substanz oder der Substanzen XAY über diese reaktiven lipophilen molekularen Bereiche miteinander intermolekulare Reaktionen eingehen können, folgen in einfachen, aber dennoch äußerst vorteilhaften Ausführungsformen Strukturschemata wie folgt:

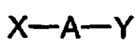

(1)

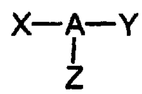

(2)

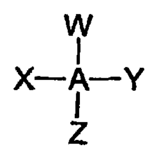

(3)

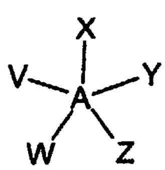

(4)

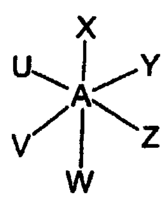

(5)

A stellt dabei jeweils einen hydrophilen, die anderen Molekülbereiche miteinander verbrückenden Molekülbereich dar.U, V, W, X, Y und Z können unabhängig voneinander gleiche oder verschiedene reaktive Molekülreste darstellen.

Intermolekulare Reaktionen beispielsweise zweier Moleküle des Molekültyps (1) können sich beispielsweise nach den folgenden Schemata abspielen:

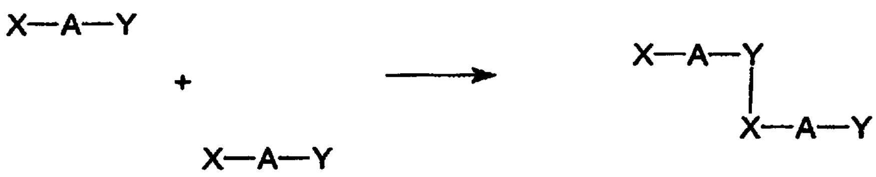

oder

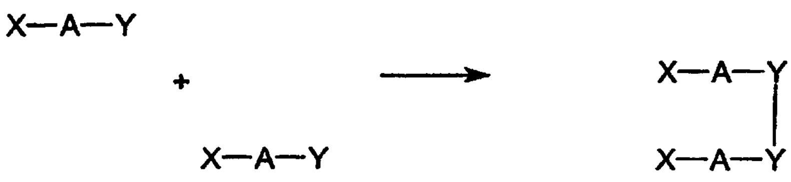

,

wobei in den Schemata unberücksichtig bleibt, welche chemische Veränderung die Molekülreste X und Y eingehen. Denkbar sind Reaktionen mit und ohne Elimination kleiner Moleküle (z.B. Wasser), Addition an Doppel- oder Dreifachbindungen, Cycloadditionen, beispielsweise vom Diels-Alder-Typ, Polymerisationsreaktionen und weiteres mehr.

Ähnliches gilt, wenn mehrere verschiedene Substanzen des Typs XAY eingesetzt werden, beispielsweise dann, wenn gleichartige Molekülreste nicht miteinander reagieren. Im nachfolgenden Schema wurde angenommen, daß X und Y verschiedene Molekülreste darstellen:

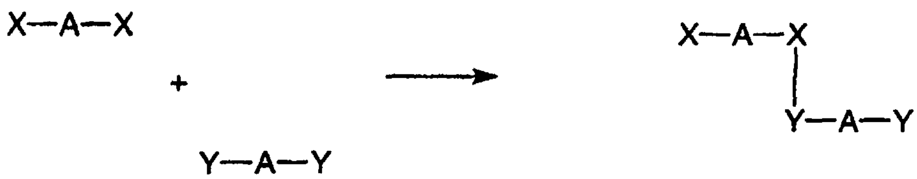

Es ist erfindungsgemäß vorteilhaft, wenn in den erfindungsgemäßen Mehrphasensystemen wenigstens einer der Reste W, X, Y und Z eine oder mehrere Doppel- und/oder Dreifachbindungen enthält und wenigstens einer der verbleibenden Reste geeignet ist, mit dem die Doppel- bzw. Dreifachbindungen tragenden Rest Reaktionen einzugehen.

Beispielsweise kann einer der Reste W, X, Y und Z eine einzelne Doppelbindung und wenigstens einer der verbleibenden Reste ebenfalls eine Doppelbindung enthalten, dergestalt, daß im Sinne einer 2 + 2 -Cycloaddition eine Reaktion gemäß dem nachstehenden Reaktionsschema ermöglicht wird.

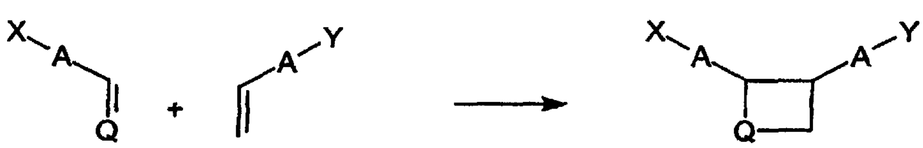

Beispielsweise kann ferner einer der Reste W, X, Y und Z eine einzelne Doppelbindung und wenigstens einer der verbleibenden Reste ein System aus konjugierten Doppelbindungen enthalten, dergestalt, daß im Sinne einer 2 + 4 - Cycloaddition eine Reaktion gemäß dem nachstehenden Reaktionsschema ermöglicht wird.

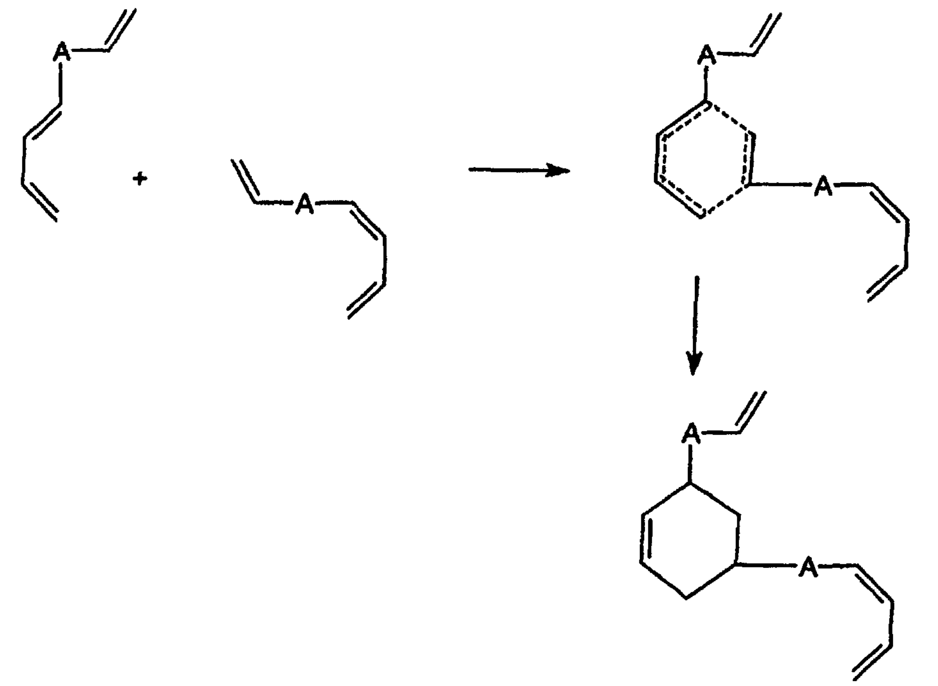

Auch andere Reaktionstypen sind erfindungsgemäß vorteilhaft, wie beispielsweise

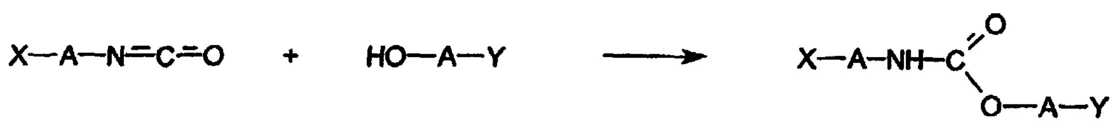

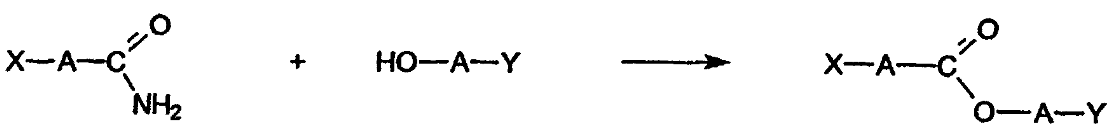

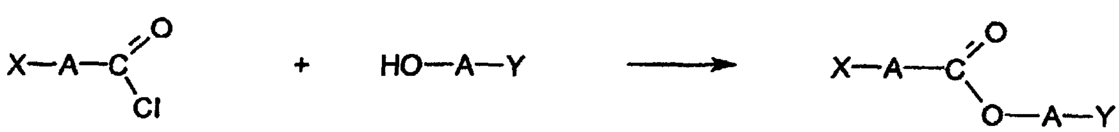

Ferner können beispielsweise einer der Reste W, X, Y und Z eine einzelne Doppelbindung und wenigstens einer der verbleibenden Reste ebenfalls eine Doppelbindung enthalten, dergestalt, daß eine Oligo- bzw. Polymerisationsreaktion gemäß dem nachstehenden Reaktionsschema ermöglicht wird.

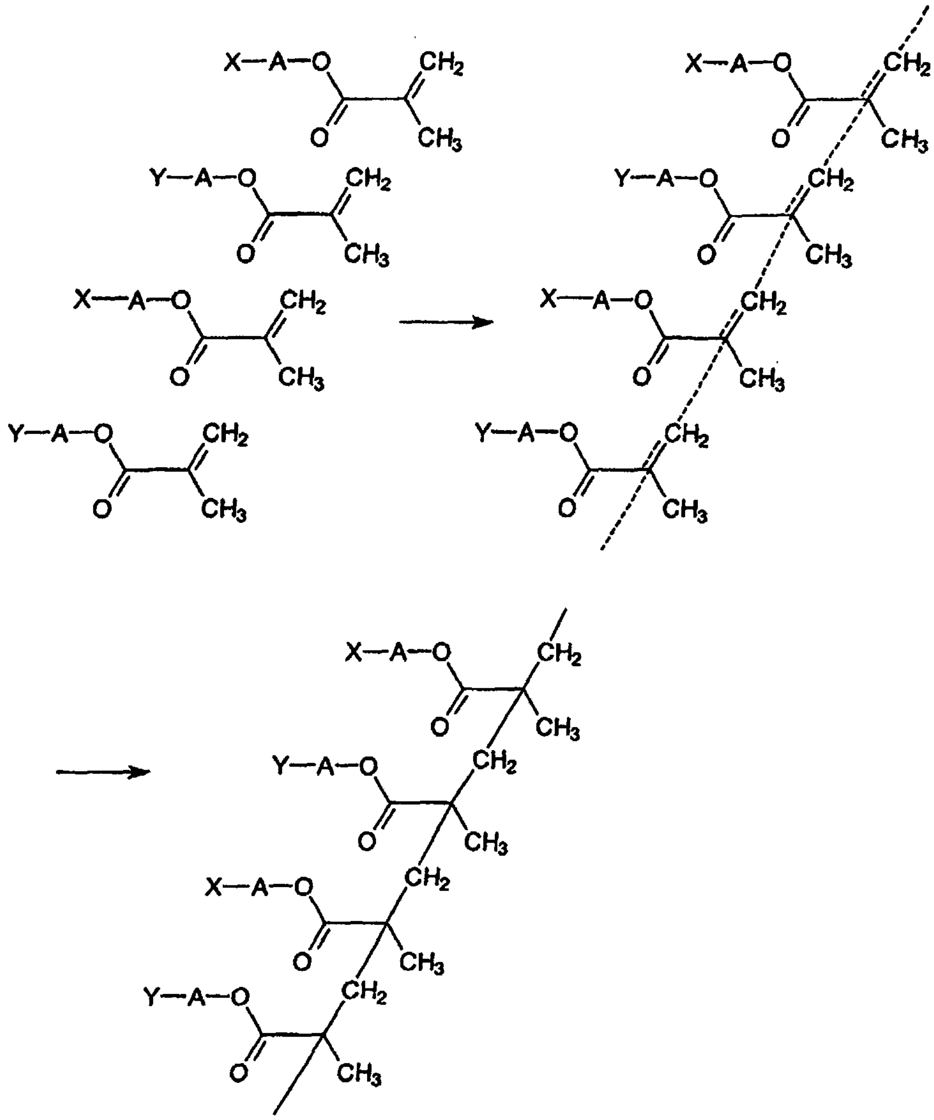

In Fig. 2 ist verdeutlicht, wie erfindungsgemäß ein die einzelnen Partikel der dispersen lipoiden Phase bzw. der dispersen lipoiden Phasen miteinander verknüpfendes makromolekulares Netzwerk zustande kommt. Dargestellt sind zwei von einer Emulgatorhülle umgebene Lipidtröpfchen, welche in einer Wasserphase dispergiert sind. Ein Molekül vom Typ XAY, taucht mit seinen lipophilen reaktiven Molekülbereichen X und Y ins Innere beider Lipidtröpfchen ein. Zwei weitere Moleküle vom Typ XAY tauchen mit lediglich einem ihrer lipophilen reaktiven Molekülbereiche ins Innere der Lipidtröpchen ein. Durch Reaktion des reaktiven Molekülbereiches X des beide Lipidtröpfchen verbrückenden Moleküls vom Typ XAY mit einem reaktiven Molekülbereich Y des zweiten Moleküls XAY und durch Reaktion des reaktiven Molekülbereiches Y des beide Lipidtröpfchen verbrückenden Moleküls vom Typ XAY mit einem reaktiven Motekülbereich X des dritten Moleküls XAY entsteht ein Kettenmolekül X-A-Y-X-A-Y-X-A-Y, wobei unberücksichtig bleibt, welche chemische Veränderung die Molekülreste X und Y eingehen. Dies ist in Fig. 3 versinnbildlicht. Die Lipophilie der Molekülbereiche X und Y, welche nach der chemischen Reaktion in der Regel keine wesentlichen Veränderungen durchlaufen hat, bewirkt, daß die Lipidtröpfchen an dem im Entstehen begriffenen Netzwerk fixiert werden.

In Fig. 4 ist ein von einer Emulgatorhülle umgebenes Lipidtröpfchen dargestellt, in welches die reaktive Bereiche X bzw. Y vierer Moleküle vom Typ XAY eintauchen. Im vorliegenden Falle wurde angenommen, daß die Molekülbereiche X und Y identisch sein sollen und durch den Acrylsäurerest repräsentiert werden - wie in den vergrößerten Bildausschnitten verdeutlicht wird. Durch Einwirkung von ultraviolettem Licht reagieren die Acrylatgruppen miteinander im Sinne einer Oligomerisation - wobei die Absättigung der endständigen Reaktandengruppen hier offengelassen wird. Die nicht in ein Lipidtröpfchen eintauchenden Acrylatgruppen nehmen hier, unter den gegebenen Umständen, nicht an Oligomerisations- oder Additionsreaktionen teil.

In Fig. 5 ist eine Emulsion versinnbildlicht, deren Lipidtröpfchen an den Vemetzungspunkten eines Netzwerkes

fixiert sind, welches durch Polymerisation einer Substanz des Typs XAY zuwege gekommen ist. Im vorliegenden Falle wurde angenommen, daß die Molekülbereiche X und Y identisch sein sollen und durch den Acrylsäurerest repräsentiert werden - wie in den vergrößerten Bildausschnitten verdeutlicht wird. Es ist von statistischen Parametern abhängig, wie viele Einzelreaktionen sich in der einzelnen Lipidpartikel abspielen. Dem wurde versucht Rechnung zu tragen, indem mal zwei, mal drei aber auch gelegentlich vier oder fünf Acrylatreste sich innerhalb einer Lipidpartikel zu einer "Oligomerengruppe" aggregieren. Der Vernetzungsgrad bestimmt letztendlich die Festigkeit des Netzwerkes. Er ist in erster Linie abhängig von der Konzentration der Substanz XAY.

Es ist erfindungsgemäß besonders vorteilhaft, wenn mindestens eine der reaktiven lipophilen molekularen Bereiche gewählt wird oder werden aus der Gruppe der Doppel- und/oder Dreifachbindungen enthaltenden Molekülreste, zum Beispiel ungesättigte Carbonsäurereste, Alkohole, Ketone, Ester, Schiffsche Basen, Aldehyde, Azoverbindungen.

Insbesondere vorteilhaft sind Mehrphasensysteme die dadurch gekennzeichnet sind, daß mindestens einer der reaktiven lipophilen molekularen Bereiche gewählt wird aus der Gruppe der Molekülreste gemäß der nachfolgenden Auflistung, in der B die Gesamtheit aus verbrückendem hydrophilem Molekülbereich und dem oder den übrigen reaktiven lipophilen molekularen Bereichen repräsentiert:

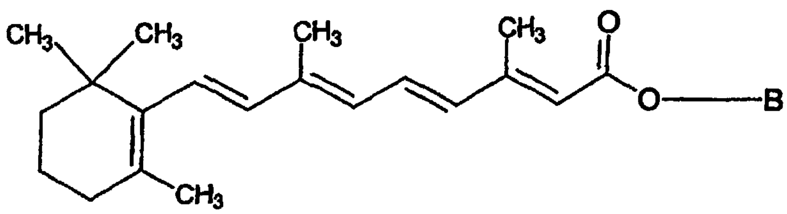

Retinsäure

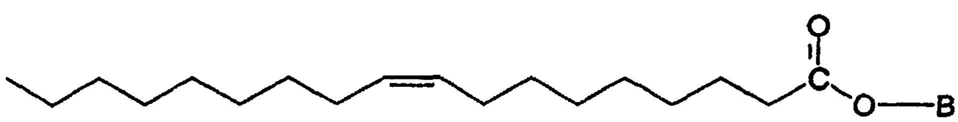

Ölsäure

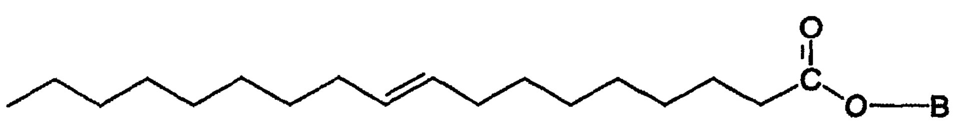

Elaidinsäure

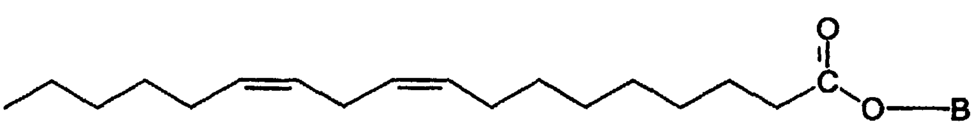

Linolsäure

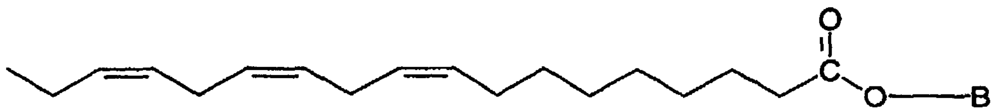

Linolensäure

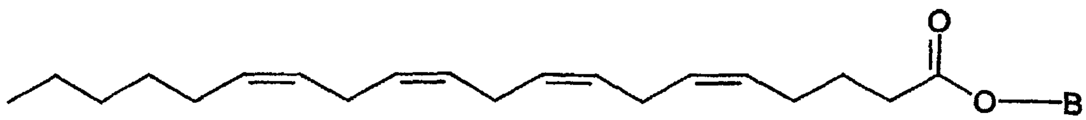

Arachidonsäure

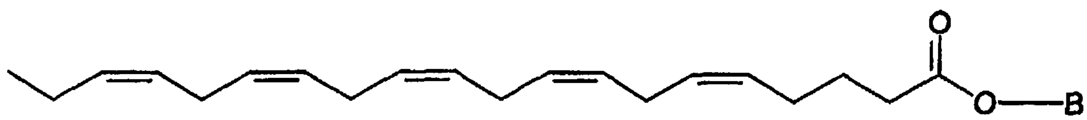

Eicosapentaënsäure

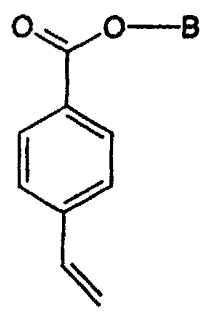

4-Vinylbenzoësäure

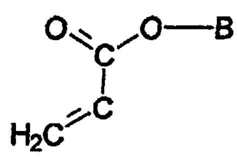

Acrylsäure

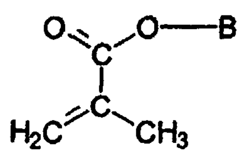

Methacrylsäure

$HC\equiv C-C\equiv C-B$

Diacetylen

$H_2C{=}CH{-}CH{=}CH{-}B$

Butadien

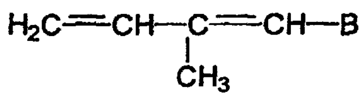

Isopren

wobei einer aber auch gewünschtenfalls mehrere bzw. alle der reaktiven lipophilen molekularen Bereiche der einzusetzenden Substanz XAY aus der Gruppe der Doppel- und/oder Dreifachbindungen enthaltenden Molekülreste, insbesondere vorteilhaft gewählt werden aus der Gruppe der Molekülreste gemäß der vorstehenden Auflistung und die reaktiven lipophilen molekularen Bereiche Innerhalb eines Moleküls gleich oder verschieden sein können.

Insbesondere, wenn die einzusetzenden Substanz XAY oder mehrere der einzusetzenden Substanzen XAY einen oder mehrere reaktive lipophile molekulare Bereiche gewählt aus der Gruppe der Doppel- und/oder Dreifachbindungen enthaltenden Molekülreste enthält, kann es vorteilhaft sein, wenigstens einen anderen reaktiven Molekülrest aus der Gruppe der Molekülreste zu wählen, welche zwar keine Doppel- und/oder Dreifachbindungen enthalten, dafür aber imstande sind mit solchen Doppel- und/oder Dreifachbindungen Reaktionen einzugehen, z.B. 4-Aminobenzoesäure, 4-Methoxyzimtsäure, 2-Hydroxy-4-methoxybenzophenon-5-sulfonsäure.

Vorteilhafte erfindungsgemäße Mehrphasensysteme zeichnen sich auch dadurch aus, daß der hydrophile Molekülbereich so gewählt wird, daß die Substanz XAY insgesamt wasserlöslich oder zumindest in Wasser dispergierbar ist.

Auch ist es für erfindungsgemäße Mehrphasensysteme vorteilhaft, wenn für das Strukturschema des hydrophilen Molekülbereiches A folgende speziellere Strukturschemata befolgt werden:

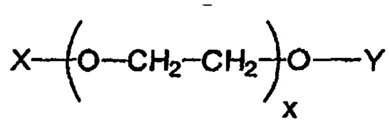

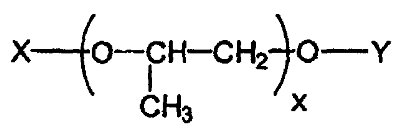

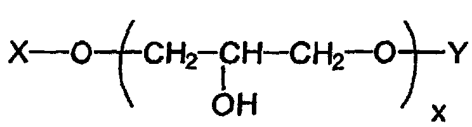

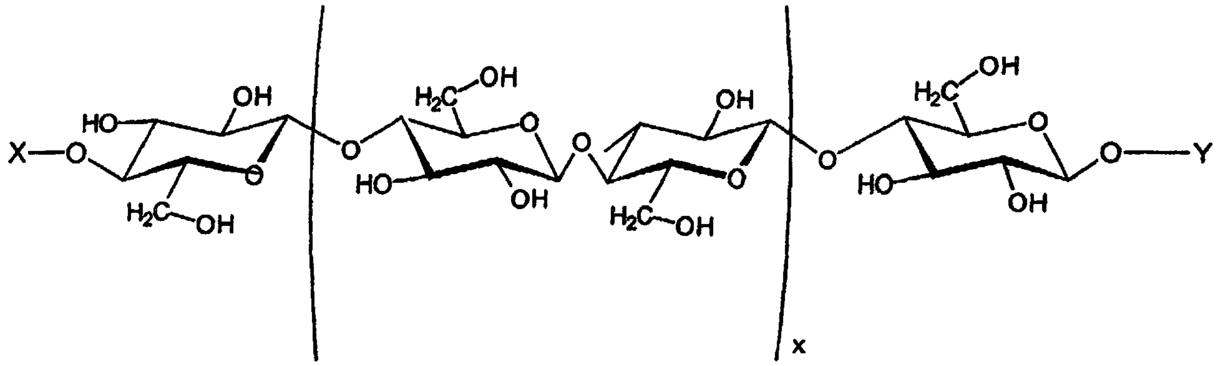

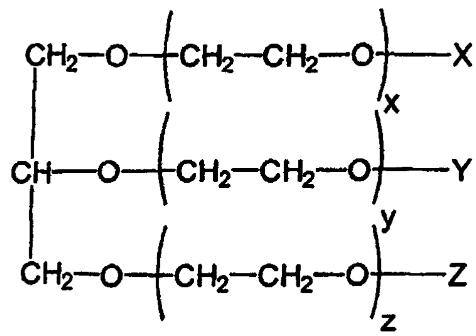

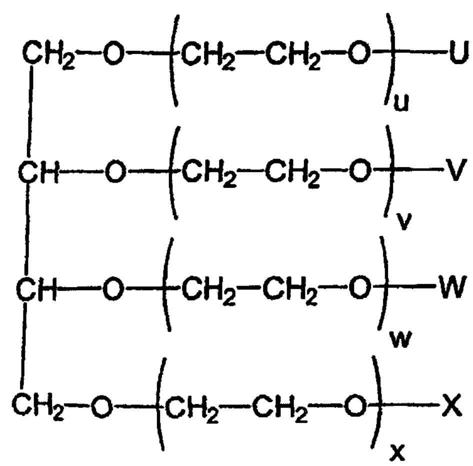

,

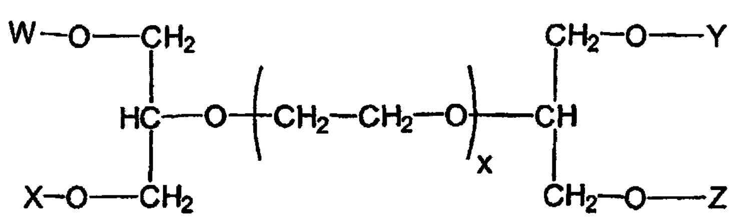

,

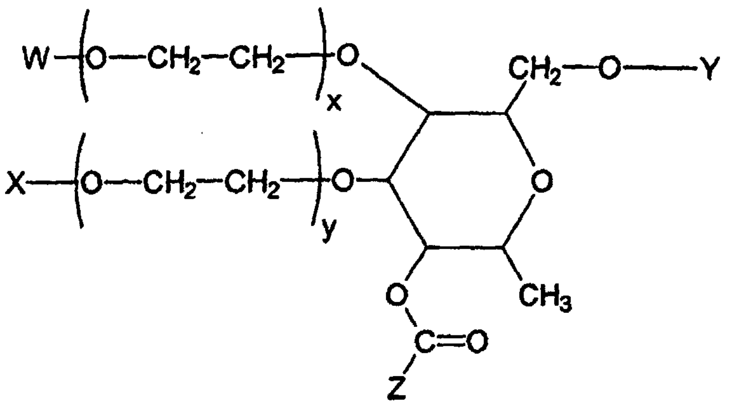

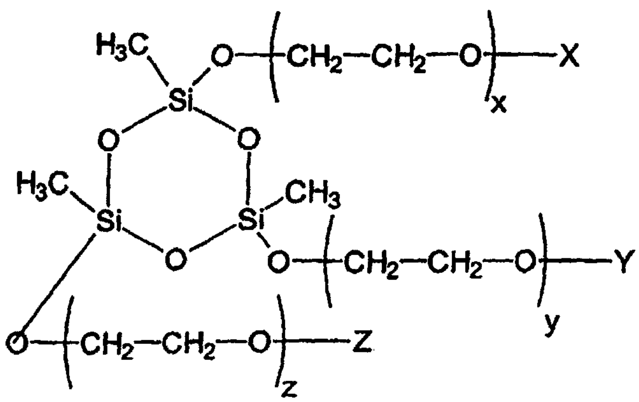

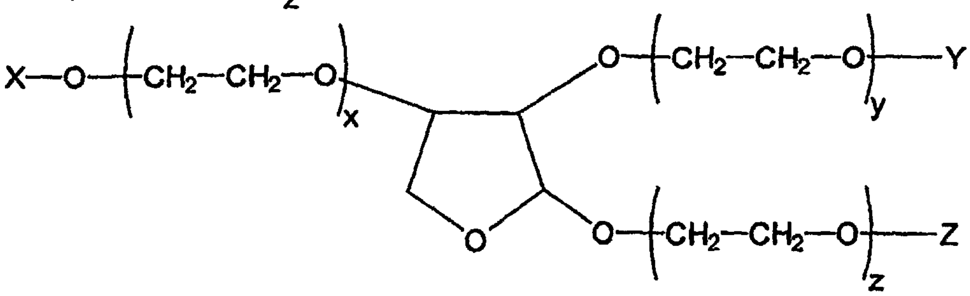

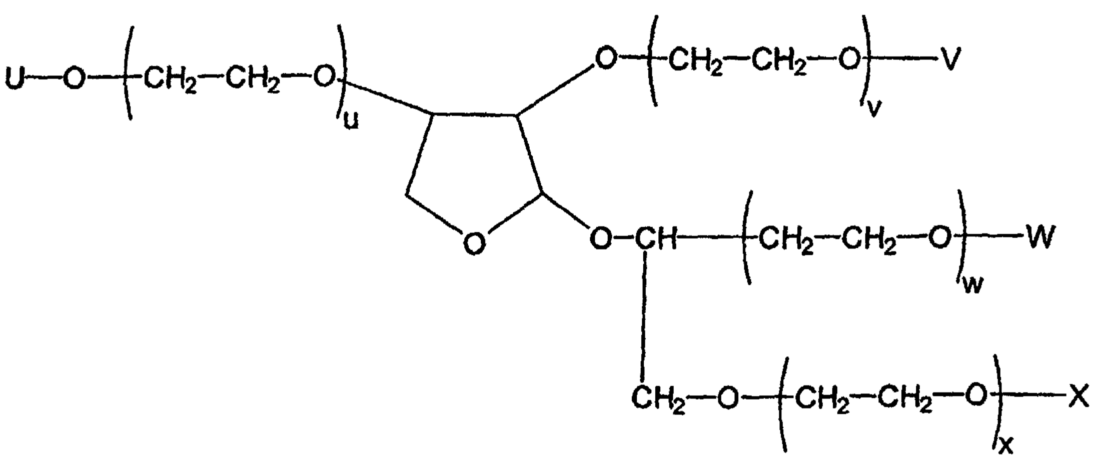

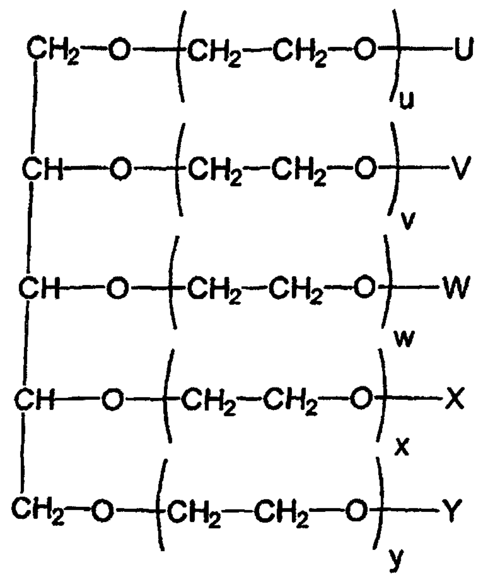

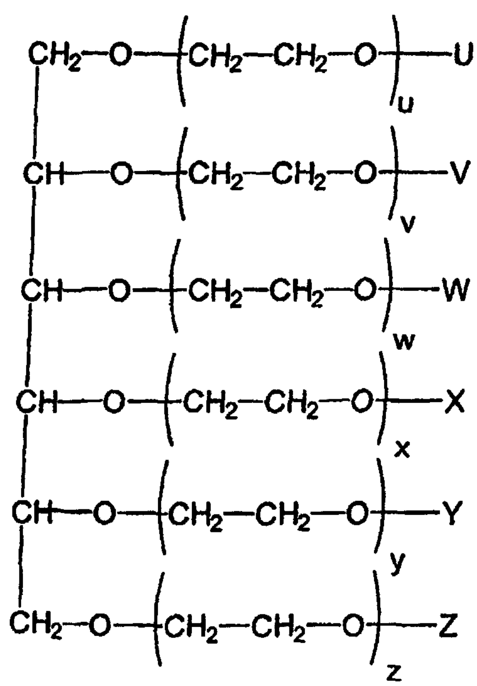

wobei U, V, W, X, Y und Z unabhängig voneinander lipophile molekulare Bereiche darstellen können, u, v, w, x, y und z dabei unabhängig voneinander Zahlen bedeuten, die es dem Gesamtmolekül erlauben, in Wasser löslich oder zumindest dispergierbar zu sein, typischerweise gewählt aus dem Bereich größer als 10, vorteilhaft aus dem Bereich 20 - 100000. Auch Teilsubstitution ist dabei erfindungsgemäß vorteilhaft möglich, wobei einer oder mehrere der Indices u, v, w, x, y, oder z den Wert Null annehmen können und einer oder mehrere der Reste U, V, W, X, Yoder Z Wasserstoffatome darstellen können.

Erfindungsgemäß vorteilhafte Mehrphasensysteme können auch dadurch gekennzeichnet sein, daß als Substanzen des Typs XAY Dicholesterylverbindungen des Typs

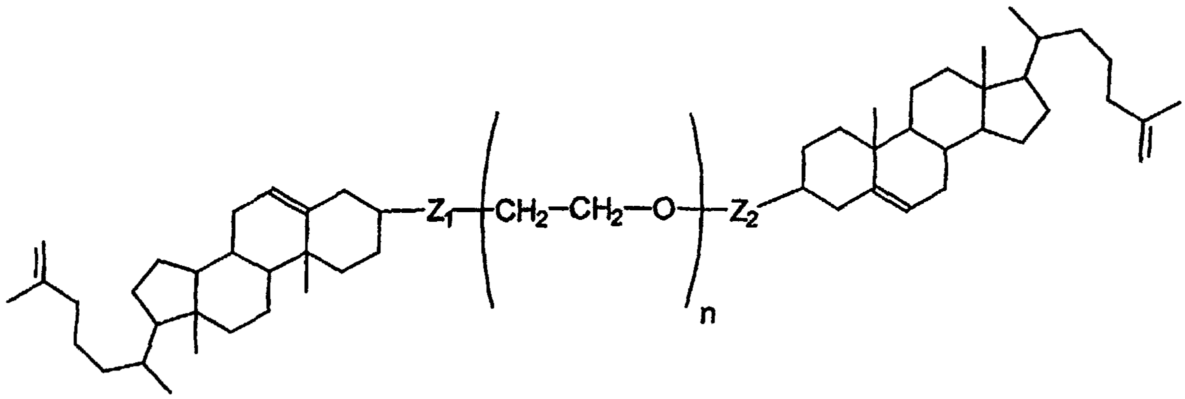

gewählt werden, wobei $Z_1$ und $Z_2$ unabhängig voneinander gewählt werden können aus der Gruppe Einfachbindung, Estergruppe, Kohlensäureestergruppe, Sauerstoff, Säureamidgruppe, Säureimidgruppe, Thiocarbonsäureestergruppe, Urethan- bzw. Carbamatgruppe.

Besonders vorteilhafte erfindungsgemäße Mehrphasensysteme zeichnen sich auch dadurch aus, daß als Substanzen des Typs XAY Dicholesterylverbindungen des Typs

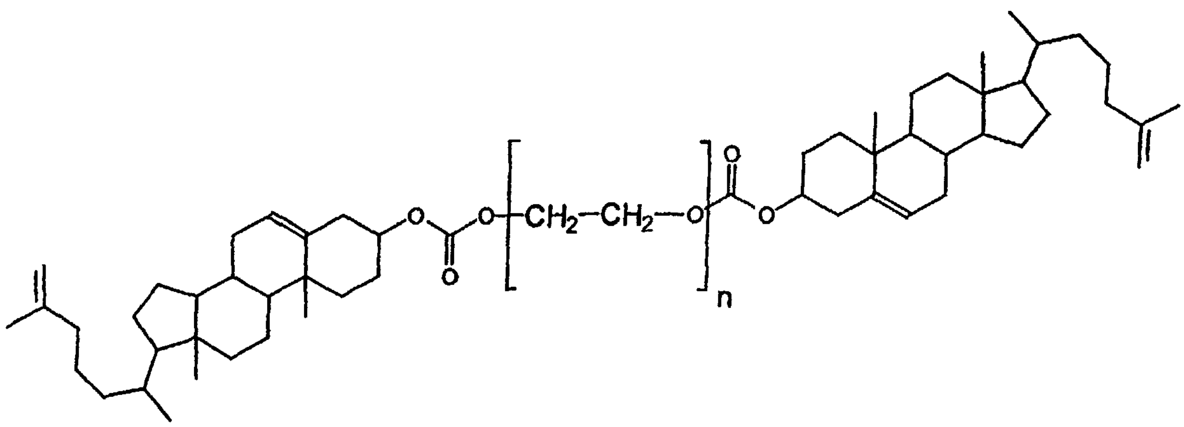

gewählt werden, welche wir kollektiv PEG-n-$Chol_2$ nennen wollen, wobei n Zahlen bedeutet, die es dem Gesamtmolekül erlaubt, in Wasser löslich oder zumindest dispergierbar zu sein, typischerweise gewählt aus dem Bereich größer als 10, vorteilhaft aus dem Bereich 20 bis $10^7$, ganz besonders vorteilhaft aus dem Bereich 120 bis 800.

PEG-n-$Chol_2$ ist nach den üblichen chemischen Verfahren erhältlich. Insbesondere vorteilhaft kann PEG-n-$Chol_2$ erhalten werden, indem Polyethylenoxid mit dem gewünschten Polymerisierungsgrade n unter mit einem Cholesterylderivat der allgemeinen Struktur

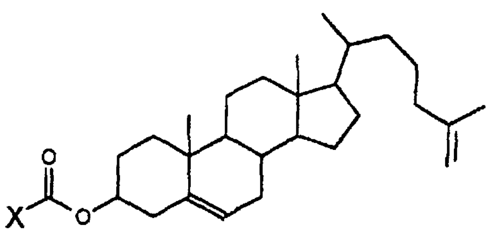

umgesetzt werden, wobei es von Vorteil ist, Reaktionsbedingungen zu schaffen, welche die Abspaltung der Substanz HX begünstigen, etwa nach dem folgenden Reaktionsschema:

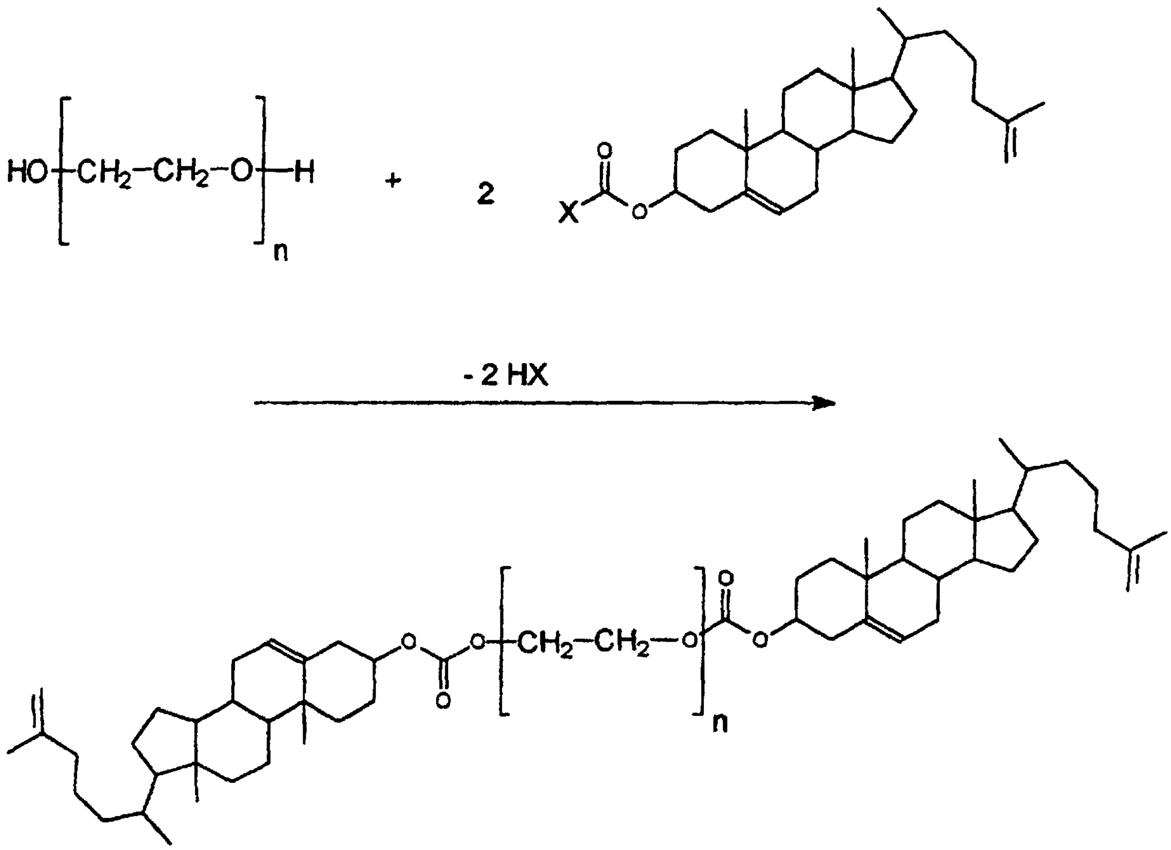


Die Menge der erfindungsgemäßen Verdicker sollte vorzugsweise im Bereich von 0,3 bis 50 Gew.-%, insbesondere 0,5 bis 30 Gew.-% liegen, jeweils bezogen auf das Gesamtgewicht des Mikroemulsionselastomers.

Beispielsweise vorteilhaft kann ebenfalls mit Linolensäure modifizierte Hydroxyethylcellulose verwendet werden.

Ganz besonders vorteilhaft im Sinne der vorliegenden Erfindung sind Polyethylenglycol-Diacrylate sowie Polyethylenglycol-Dimethacrylate des Typs

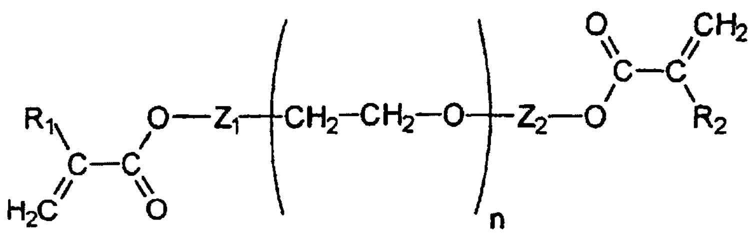
,

wobei n Zahlen bedeutet, die es dem Gesamtmolekül erlaubt, in Wasser löslich oder zumindest dispergierbar zu sein, typischerweise gewählt aus dem Bereich größer als 10, vorteilhaft aus dem Bereich 20 bis $10^7$, ganz besonders vorteilhaft aus dem Bereich 120 bis 800, und wobei $Z_1$ und $Z_2$ unabhängig voneinander gewählt werden können aus der Gruppe Einfachbindung, Estergruppe, Kohlensäureestergruppe, Sauerstoff, Säureamidgruppe, Säureimidgruppe, Thiocarbonsäureestergruppe, Urethan- bzw. Carbamatgruppe. $R_1$ und $R_2$ bedeuten unabhängig voneinander H oder Methyl.

In einer weiteren vorteilhaften Ausführungsform können $Z_1$ und $Z_2$ weitere organische Gruppen wie beispielsweise die Gruppe

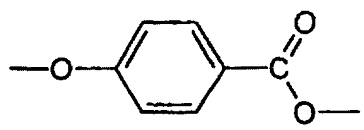

darstellen, so daß eine konkrete vorteilhafte Ausführungsform durch die Struktur

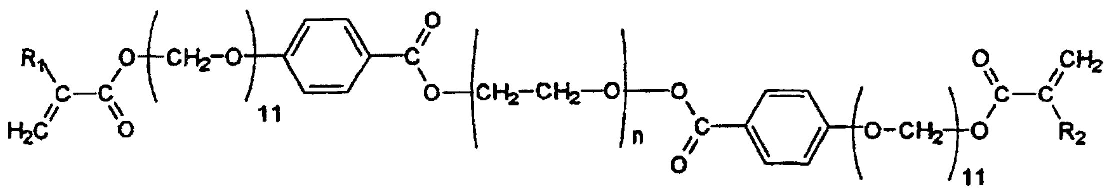

gegeben ist, wobei n Zahlen bedeutet, die es dem Gesamtmolekül erlaubt, in Wasser löslich oder zumindest dispergierbar zu sein, typischerweise gewählt aus dem Bereich größer als 10, vorteilhaft aus dem Bereich 20 bis $10^7$, ganz besonders vorteilhaft aus dem Bereich 120 bis 800.

Vorteilhaft sind auch folgende Verbindungstypen:

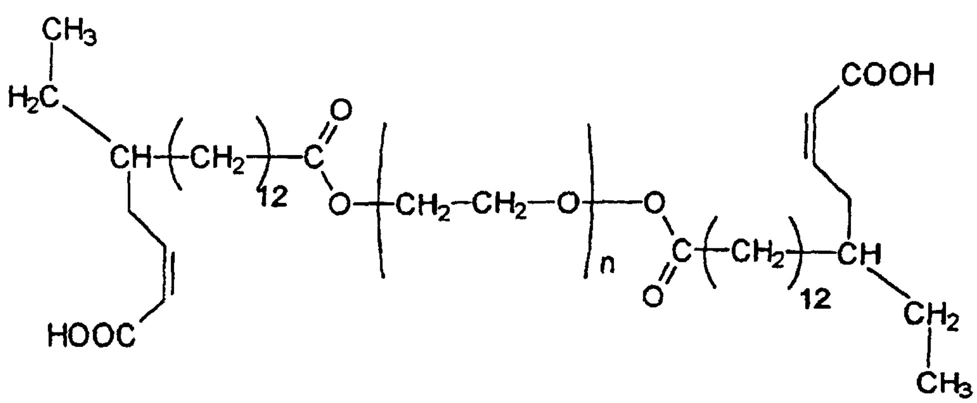

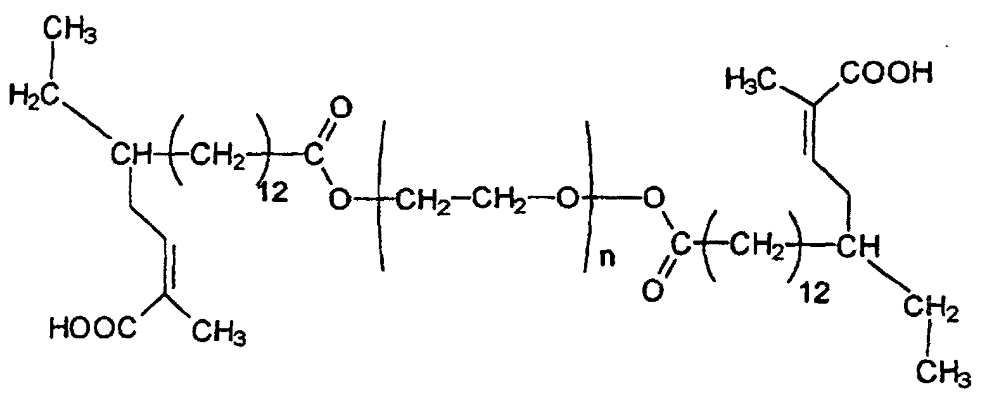

wobei n Zahlen bedeutet, die es dem Gesamtmolekül erlaubt, in Wasser löslich oder zumindest dispergierbar zu sein, typischerweise gewählt aus dem Bereich größer als 10, vorteilhaft aus dem Bereich 20 bis $10^7$, ganz besonders vorteilhaft aus dem Bereich 120 bis 800.

Den erfindungsgemäßen Zusammensetzungen liegen vorteilhaft O/W-Makroemulsionen, O/W-Mikroemulsionen, W/O/W-Emulsionen oder Hydrodispersionen zugrunde. Auch andere Formulierungstypen wie Dispersionen von Festkörperpartikeln (z.B. sogenannte "Solid-lipid-Nanoparts") sind im Sinne der vorliegenden Erfindung vorteilhaft.

Vorteilhaft werden die erfindungsgemäßen Zusammensetzungen erhalten, indem zunächst das zugrundeliegende disperse. Mehrphasensystem auf dem Fachmanne an sich geläufigem Wege hergestellt wird und hernach die Substanz oder die Substanzen des Typs XAY zugegeben wird oder werden.

Es war erstaunlich, daß allein durch zugeführte Wärme O/W-Makroemulsionen, O/W-Mikroemulsionen, W/O/W-Emulsionen, Festkörperdispersionen oder Hydrodispersionen nach Zusatz von Vernetzermolekülen, die ungesättigte hydrophobe Gruppen tragen, mittels kovalent verknüpfter Netzwerke vernetzte, disperse Zwei- oder Mehrphasensysteme über einen sehr großen Temperaturbereich hergestellt werden können, ohne daß Monomere oder andere chemische Zusätze eingesetzt werden müssen.

So erlaubt das Verfahren der Herstellung dieser mittels kovalent verknüpfter Netzwerke vernetzte, disperse Zwei- oder Mehrphasensysteme, daß man beispielsweise eine eine Mikroemulsion in Gegenwart der Polymere nach der Phaseninversion bei 60°C solange hält, bis die sich erfindungsgemäße Mikroemulsionselastomere bilden. Es ist unerheblich, ob das Polymer die Phaseninversion ebenfalls durchläuft oder erst nachträglich zur Mikroemulsion zugesetzt wird. Ferner können auch Mischungen aus Polymeren eingesetzt werden, die es erlauben kovalente und physikalisch vemetzte Mikroemulsionen zu erzeugen.

Auf diese Weise ergibt sich eine enorme Vielfalt von Mikroemulsionselastomeren, die es erlauben, für den beabsichtigten Anwendungszweck maßgeschneiderte Produkte mit der gewünschten Konsistenz herzustellen (Chromatographie, Katalysatorsysteme, Kontaktlinsen, Implantante, Wundauflagen, Trägersysteme für transdermale Darreichungen, Membranen, Klebebänder).

So können beispielsweise Materialien hergestellt werden, die am Polymer kovalent gebundene Lichtschutzfilter oder Farbstoffe enthalten oder auch in die Mikroemulsionselastomere eingearbeite Verbindungen, die als Lichtschutzfilter oder Farbstoffe bekannt sind (Sonnenschirme auf Basis eines Mikroemulsionselastomers zum Beispiel).

Sollen beispielsweise aus Umweltschutzgründen nur hoch verträgliche Polymere eingesetzt werden, kann man Polymere auf Basis von Dextranen, Polysacchariden, Alginaten Chitin, Hyaluronsäure Polyglycolsäure, Polyorthoester usw. verwenden, die mit gut biologisch abbaubaren hydrophoben Gruppen (Vitamin-A-Alkohole, Urethane, Linolsäure, Linolensäure, Ölsäure, Polylactide usw.) substituiert werden, die selbst unbedenklich sind und zum Beispiel enzymatisch oder durch Hydrolyse wieder in die Ausgangsverbindungen gespalten werden. Ähnliche Prinzipien wird der Fachmann verfolgen, wenn er an medizinischen Anwendungen interessiert ist.

Das Verfahren kann auch oberhalb oder innerhalb der Phaseninversion durchgeführt werden, wo die Mikroemulsionen in andere Phasen übergehen, sodaß zum Beispiel vernetzte flüssig-kristalline oder bikontinuierliche Mikroemulsionselastomere entstehen. Diese Systeme können dann ein opakes oder weißes Aussehen haben.

Das vorab beschriebene Verfahren ist auch auf photochemischem Wege durchführbar. Man kann zum Beispiel diese Elastomere durch Bestrahlen der mit Polymer zunächst physikalisch vemetzten Mikroemulsion mit einer Quecksilberdampflampe erhalten. Ferner können auch für Wirkstoffe oder Farbstoffe auf diese Weise mit in die Mikroemulsionselastomere eingeschlossen werden.

Je nach Verwendungszweck enthalten die erfindungsgemäßen Zusammensetzungen übliche Bestandteile und Inhaltsstoffe.

Sofern den erfindungsgemäßen dispersen Mehrphasensysteme beispielsweise O/W-Makroemulsionen, oder O/W-Mikroemulsionen zugrundeliegen, enthalten diese als Grundbestandteile Wasser Öl sowie einen O/W-Emulgator bzw. ein O/W-Emulgatorsystem, welches gewünschtenfalls zusätzlich W/O-Emulgatoren enthalten kann, ferner übliche Hilfs- Zusatz- und/oder Wirkstoffe.

Gelartige Zubereitungen für kosmetische oder dermatologische Zwecke enthalten selbstverständlich nur Bestandteile, welche kosmetisch bzw. physiologisch unbedenklich sind.

Vorteilhaft werden der polyethoxylierte bzw. polypropoxylierte bzw. polyethoxylierte und polypropoxylierte O/W-Emulgator oder die polyethoxylierten bzw. polypropoxylierten bzw. polyethoxylierten und polypropoxylierten O/W-Emulgatoren verwendet, welche gewählt werden können aus der Gruppe

- der Fettalkoholethoxylate der allgemeinen Formel $R-O-(-CH_2-CH_2-O-)_n-H$, wobei R einen verzweigten oder unverzweigten Alkyl- oder Alkenylrest und n eine Zahl von 10 bis 50 darstellen
- der ethoxylierten Wollwachsalkohole,
- der Polyethylenglycolether der allgemeinen Formel $R-O-(-CH_2-CH_2-O-)_n-R'$, wobei R und R' unabhängig voneinander verzweigte oder unverzweigte Alkyl- oder Alkenylreste und n eine Zahl von 10 bis 80 darstellen
- der Fettsäureethoxylate der allgemeinen Formel $R-COO-(-CH_2-CH_2-O-)_n-H$, wobei R einen verzweigten oder unverzweigten Alkyl- oder Alkenylrest und n eine Zahl von 10 bis 40 darstellen,
- der veretherten Fettsäureethoxylate der allgemeinen Formel $R-COO-(-CH_2-CH_2-O-)_n-R'$, wobei R und R' unabhängig voneinander verzweigte oder unverzweigte Alkyl- oder Alkenylreste und n eine Zahl von 10 bis 80 darstellen.
- der veresterten Fettsäureethoxylate der allgemeinen Formel $R-COO-(-CH_2-CH_2-O-)_n-C(O)-R'$, wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweiger Fettsäuren und einem Ethoxylierungsgrad zwischen 3 und 50,

- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- derAlkylethercarbonsäuren der allgemeinen Formel $R-O-(-CH_2-CH_2-O-)_n-CH_2-COOH$ bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel $R-O-(-CH_2-CH_2-O-)_n-SO_3-H$ mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen.
- der Fettalkoholpropoxylate der allgemeinen Formel $R-O-(-CH_2-CH(CH_3)-O-)_n-H$, wobei R einen verzweigten oder unverzweigten Alkyl- oder Alkenylrest und n eine Zahl von 10 bis 80 darstellen,
- der Polypropylenglycolether der allgemeinen Formel $R-O-(-CH_2-CH(CH_3)-O-)_n-R'$, wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel $R-COO-(-CH_2-CH(CH_3)-O-)_n-R'$, wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel $R-COO-(-CH_2-CH(CH_3)-O-)_n-C(O)-R'$, wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel $R-COO-(-CH_2-CH(CH_3)-O-)_n-H$, wobei R einen verzweigten oder unverzweigten Alkyl- oder Alkenylrest und n eine Zahl von 10 bis 80 darstellen,
- der Polypropylenglycolglycerinfettsäureester gesättigter und/oder ungesättigter, verzweigter und/oder unverzweiger Fettsäuren und einem Propoxylierungsgrad zwischen 3 und 80
- der propoxylierten Sorbitanester mit einem Propoxylierungsgrad von 3 bis 100
- der Cholesterinpropoxylate mit einem Propoxylierungsgrad von 3 bis 100
- der propoxylierten Triglyceride mit einem Propoxylierungsgrad von 3 bis 100
- der Alkylethercarbonsäuren der allgemeinen Formel $R-O-(-CH_2-CH(CH_3)O-)_n-CH_2-COOH$ bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel $R-O-(-CH_2-CH(CH_3)-O-)_n-SO_3-H$ mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylate/propoxylate der allgemeinen Formel $R-O-X_n-Y_m-H$, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel $R-O-X_n-Y_m-R'$, wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel $R-COO-X_n-Y_m-R'$, wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel $R-COO-X_n-Y_m-H$, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

Insbesondere ist vorteilhaft, wenn der polyethoxylierte bzw. polypropoxylierte bzw. polyethoxylierte und polypropoxylierte O/W-Emulgator oder die polyethoxylierten bzw. polypropoxylierten bzw. polyethoxylierten und polypropoxylierten O/W-Emulgatoren gewählt wird oder werden aus der Gruppe

- der Fettalkoholethoxylate der allgemeinen Formel $R-O-(-CH_2-CH_2-O-)_n-H$, wobei R einen verzweigten oder unverzweigten Alkyl- oder Alkenylrest mit 5 - 30 C-Atomen und n eine Zahl von 10 bis 25 darstellen

- der ethoxylierten Wollwachsalkohole mit HLB-Werten von 11 - 16, ganz besonders vorteilhaft mit mit HLB-Werten von 14,5 - 15,5.
- der Polyethylenglycolether der allgemeinen Formel $R-O-(-CH_2-CH_2-O-)_n-R'$, wobei R und R' unabhängig voneinander verzweigte oder unverzweigte Alkyl- oder Alkenylreste mit 5 - 30 C-Atomen und n eine Zahl von 10 bis 25 darstellen,
- der Fettsäureethoxylate der allgemeinen Formel $R-COO-(-CH_2-CH_2-O-)_n$ -H, wobei R einen verzweigten oder unverzweigten Alkyl- oder Alkenylrest mit 5 - 30 C-Atomen und n eine Zahl von 10 bis 25 darstellen,
- der veretherten Fettsäureethoxylate der allgemeinen Formel $R-COO-(-CH_2-CH_2-O-)_n-R'$, wobei R und R' unabhängig voneinander verzweigte oder unverzweigte Alkyl- oder Alkenylreste mit 5 - 30 C-Atomen und n eine Zahl von 10 bis 50 darstellen,
- der veresterten Fettsäureethoxylate der allgemeinen Formel $R-COO-(-CH_2-CH_2-O-)_n$ -C(O)-R', wobei R und R' unabhängig voneinander verzweigte oder unverzweigte Alkyl- oder Alkenylreste mit 5 - 30 C-Atomen und n eine Zahl von 10 bis 50 darstellen,
- der Polyethylenglycolglycerinfettsäureester gesättigter und/oder ungesättigter, verzweigter und/oder unverzweigter Fettsäuren mit 6 bis 26 C-Atomen und einem Ethoxylierungsgrad zwischen 3 und 50
- der ethoxylierten Sorbitanester mit einem Ethoxylierungsgrad von 3 bis 30
- der Cholesterinethoxylate mit HLB-Werten von 11 - 16, ganz besonders vorteilhaft mit HLB-Werten von 14,5 - 15,5
- der ethoxylierten Triglyceride mit HLB-Werten von 11 - 16, ganz besonders vorteilhaft mit mit HLB-Werten von 14,5 - 15,5
- der Alkylethercarbonsäuren der allgemeinen Formel $R-O-(-CH_2-CH_2-O)_n-CH_2-COOH$ bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 20 darstellen,
- der Polyoxyethylensorbitolfettsäureester, basierend auf verzweigten oder unverzweigten Alkan- oder Alkensäuren und einen Ethoxylierungsgrad von 10 bis 80 aufweisend, beispielsweise vom Sorbeth-Typ,
- der Alkylethersulfate bzw. die diesen Sulfaten zugrundeliegenden Säuren der allgemeinen Formel $R-O-(-CH_2-CH_2-O-)_n$ $-SO_3-H$ mit kosmetisch oder pharmazeutisch akzeptablen Kationen, wobei R einen verzweigten oder unverzweigten Alkyl- oder Alkenylrest mit 5 - 30 C-Atomen und n eine Zahl von 3 bis 30 darstellen,
- der Fettalkoholpropoxylate der allgemeinen Formel $R-O-(-CH_2-CH(CH_3)-O-)_n-H$, wobei R einen verzweigten oder unverzweigten Alkyl- oder Alkenylrest mit 5 - 30 C-Atomen und n eine Zahl von 10 bis 30 darstellen,
- der Polypropylenglycolether der allgemeinen Formel $R-O-(-CH_2-CH(CH_3-O-)_n-R'$, wobei R und R' unabhängig voneinander verzweigte oder unverzweigte Alkyl- oder Alkenylreste mit 5 - 30 C-Atomen und n eine Zahl von 10 bis 40 darstellen,
- der propoxylierten Wollwachsalkohole mit HLB-Werten von 11 - 16, ganz besonders vorteilhaft mit HLB-Werten von 14,5 - 15,5,
- der Fettsäurepropoxylate der allgemeinen Formel $R-COO-(-CH_2-CH(CH_3)-O-)_n-H$, wobei R einen verzweigten oder unverzweigten Alkyl- oder Alkenylrest mit 5 - 30 C-Atomen und n eine Zahl von 10 bis 40 darstellen,
- der veretherten Fettsäurepropoxylate der allgemeinen Formel $R-COO-(-CH_2-CH(CH_3)-O-)_n-R'$, wobei R und R' unabhängig voneinander verzweigte oder unverzweigte Alkyl- oder Alkenylreste mit 5 - 30 C-Atomen und n eine Zahl von 10 bis 30 darstellen,
- der veresterten Fettsäurepropoxylate der allgemeinen Formel $R-COO-(-CH_2-CH(CH_3)-O-)_n-C(O)-R'$, wobei R und R' unabhängig voneinander verzweigte oder unverzweigte Alkyl- oder Alkenylreste mit 5 - 30 C-Atomen und n eine Zahl von 10 bis 50 darstellen,
- der Polypropylenglycolglycerinfettsäureester gesättigter und/oder ungesättigter, verzweigter und/oder unverzweigter Fettsäuren mit 6 bis 26 C-Atomen und einem Propoxylierungsgrad zwischen 3 und 30
- der propoxylierten Sorbitanester mit einem Propoxylierungsgrad von 3 bis 100
- der Cholesterinpropoxylate mit HLB-Werten von 11 - 16, ganz besonders vorteilhaft mit mit HLB-Werten von 14,5 - 15,5
- der propoxylierten Triglyceride mit HLB-Werten von 11 - 16, ganz besonders vorteilhaft mit mit HLB-Werten von 14,5 - 15,5
- der Alkylethercarbonsäuren der allgemeinen Formel $R-O-(CH_2-CH(CH_3)O-)_n-CH_2-COOH$ bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 30 darstellen,
- der Alkylethersulfate bzw. die diesen Sulfaten zugrundeliegenden Säuren der allgemeinen Formel $R-O-(-CH_2-CH(CH_3)-O-)_n-SO_3-H$ mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 30 darstellen.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 17, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(12)stearylether (Steareth-12), Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20),

Polyethylenglycol(12)cetylether (Ceteth-12), Polyethylenglycol(13)cetylether (Ceteth-13), Polyethylenglycol(14) cetylether(Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19) cetylether (Ceteth-19), Polyethylenglycol(20)cetylether (Ceteth-20),

Polyethylenglycol(12)isocetylether (Isoceteth-12), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16) isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14) oleylether (Oleth-14), Polyethylenglycol(15)oleylether (Oleth-15),

Polyethylenglycol(12)laurylether (Laureth-12), Polyethylenglycol(13)laurylether (Laureth-13), Polyethylenglycol (14)laurylether (Laureth-14), Polyethylenglycol(15)laurylether (Laureth-15), Polyethylenglycol(16)laurylether (Laureth-16), Polyethylenglycol(17)laurytether (Laureth-17), Polyethylenglycol(18)laurylether (Laureth-18), Polyethylenglycol(19)laurylether (Laureth-19), Polyethylenglycol(20)laurylether (Laureth-20),

Polyethylenglycol(12)cetylstearylether (Ceteareth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(12)stearat, Polyethylenglycol(13)stearat, Polyethylenglycol(14)stearat, Polyethylenglycol(15) stearat, Polyethylenglycol(16)stearat, Polyethylenglycol(17)stearat, Polyethylenglycol(18)stearat, Polyethylenglycol(19)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol (25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat Polyethylenglycol(12)laurat, Polyethylenglycol(13)laurat, Polyethylenglycol(14)laurat,

Polyethylenglycol(15)laurat, Polyethylenglycol(16)laurat, Polyethylenglycol(17)laurat, Polyethylenglycol(18)laurat, Polyethylenglycol(19)laurat, Polyethylenglycol(20)laurat.

Als ethoxylierte Alkylethercarbonsäure bzw. deren Salz kann vorteilhaft das Natriumlaureth-11-carboxylat verwendet werden.

Als ethoxyliertes Cholesterinderivat kann vorteilhaft Polyethylenglycol(30)Cholesterylether verwendet werden.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(15) glyceryllaurat, Polyethylenglycol(7)glycerylcocoat, Polyethylenglycol(6)glycerylcaprat, Polyethylenglycol(12)glyceryllaurat, Polyethylenglycol(20)glyceryloleat zu wählen.

Es ist ebenfalls günstig, die Sorbitanester aus der Gruppe Polyethylenglycol(5)sorbitanmonoisostearat, Polyethylenglycol(10)sorbitanmonolaurat, Polyethylenglycol(20)-sorbitanmonolaurat, Polyethylenglycol(20)sorbitanmonostearat, Polyethylenglycol(20)-sorbitanmonoisostearat, Polyethylenglycol(20)sorbitanmonopalmitat, Polyethylenglycol(20)sorbitanmonooleat zu wählen.

Der oder die Emulgatoren aus der Gruppe der Polyoxyethylen-polyoxypropylen-Copolymere und der Polyoxyethylen-polyoxypropylen-Block-Copolymere können vorteilhaft aus der Gruppe der Poloxamere genannten Substanzen gewählt werden.

Als fakultative, dennoch erfindungsgemäß vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Triglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Triglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonolsostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Es ist erfindungsgemäß möglich, den Gesamtgehalt an Emulgatoren kleiner als 15 Gew.-%, bezogen auf das Gesamtgewicht der Mikroemulsion, zu halten. Es wird bevorzugt, den Gesamtgehalt an Emulgatoren kleiner als 10 Gew.%, insbesondere kleiner als 8 Gew.-% bezogen auf das Gesamtgewicht der Mikroemulsion, zu halten.

Die Ölphase der erfindungsgemäßen Mikroemulsionen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der gesättigten und/oder ungesättigten, verzweigten und/oder unverzweigten Alkohole einer Kettenlänge von 3 bis 30 C-Atomen, der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether sowie der Fettsäuretriglyceride. Die Fettsäuretriglyceride können vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr

Auch beliebige Abmischungen solcher Öl- und Wachskomponenten sind vorteilhaft im Sinne der vorliegenden

Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind femer Mischungen aus Cyclomethicon und Isotridecylisononanoat, aus Cyclomethicon und 2-Ethylhexylisostearat.

Die Herstellung der erfindungsgemäßen Mikroemulsionen erfolgt vorteilhaft dergestalt, daß ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatzund/oder Wirkstoffe, welche unterhalb des Phaseninversionstemperaturbereiches eine O/W-Emulsion bilden, auf eine Temperatur oberhalb oder innerhalb des Phaseninversionstemperaturbereiches bringt, und die gebildete Mikroemulsion hernach auf Raumtemperatur abkühlt. Dies geschieht bevorzugt unter Rühren.

Erstaunlicherweise ist es jeweils möglich, auf einen Homogenisierungsschritt zu verzichten.

Als vorteilhafte Verkörperung der vorliegenden Erfindung wird ebenfalls angesehen ein Verfahren zur Herstellung von O/W-Mikroemulsionen, dadurch gekennzeichnet, daß eine Wasserphase, gegebenenfalls übliche, in Wasser lösliche oder dispergierbare Substanzen, eine Ölphase, welche im wesentlichen aus schwerflüchtigen Bestandteilen zusammengesetzt ist, sowie

- ein oder mehrere polyethoxylierte O/W-Emulgatoren und/oder
  ein oder mehrere polypropoxylierte O/W-Emulgatoren und/oder
  ein oder mehrere polyethoxylierte und polypropoxylierte O/W-Emulgatoren,
- gewünschtenfalls ferner ein oder mehrere W/O-Emulgatoren,

ferner gegebenenfalls weitere in der Ölphase lösliche oder dispergierbare Substanzen, zusammengegeben werden und unter Rühren ein Gemisch gebildet wird, dergestalt, daß

(a) dieses Gemisch durch geeignete Wahl der Parameter, gewählt aus der Gruppe Temperatur und der Konzentration bzw. Konzentrationen mindestens eines der gewählten Emulgatoren und/oder der Ölphase und/oder der Wasserphase, in den Phaseninversionsbereich gebracht wird, in welchem sich W/O-Emulsionen in O/W-Emulsionen bzw. O/W-Emulsionen in W/O-Emulsionen umwandeln,
(b) sodann durch Variation mindestens eines Parameters, gewählt aus der Gruppe Temperatur und der Konzentration bzw. Konzentrationen mindestens eines der gewählten Emulgatoren das so gebildete Gemisch aus dem besagten Phaseninversionsbereich heraus in den Bereich gebracht wird, wo das Gemisch als O/W-Emulsion bzw. O/W-Mikroemulsion vorliegt,
(c) das besagte Gemisch sodann gegebenenfalls weiteren Aufbereitungsschritten, insbesondere einem oder mehreren Homogenisierungsschritten unterworfen wird.

Erfindungsgemäß gleichermaßen vorteilhaft sind Verfahren, bei welchen die Variation des Parameters oder der Parameter darin besteht, daß

(a) bei vorgegebener Konzentration des O/W-Emulgators bzw. der Vielzahl an O/W-Emulgatoren die Temperatur des Gemisches variiert wird, bzw. daß
(b) bei vorgegebener Temperatur die Konzentration mindestens eines O/W-Emulgators, bzw. daß
(c) bei vorgegebener Temperatur und vorgegebener Konzentration mindestens eines O/W-Emulgators, die Konzentration der Ölphase und oder die Konzentration der Wasserphase variiert wird.

Es kann erfindungsgemäß gegebenenfalls bevorzugt sein, mehrere Parameter gleichzeitig oder nacheinander zu variieren.

Mikroemulsionen eignen sich auch für andere kosmetische dermatologische Anwendungen, beispielsweise Desodorantien, so daß die vorliegende Erfindung in einer besonderen Ausführungsform Mikroemulsionsgele oder -stifte als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Desodorantien sollen folgende Bedingungen erfüllen:

1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Auch die Verwendung von Mikroemulsionen als Grundlage für desodorierende oder antitranspirant wirkende Zubereitungen sind bekannt. Deren relativ hoher Gehalt an Emulgatoren, mit den geschilderten Nachteilen, war bisher ein Übelstand, dem es abzuhelfen galt.

Eine weitere Aufgabe der vorliegenden Erfindung war es also, Zubereitungen zu entwickeln, welche als Grundlage für kosmetische Desodorantien bzw. Antitranspirantien geeignet sind, und die Nachteile des Standes der Technik nicht aufweisen.

Weiterhin war es eine Aufgabe der Erfindung, kosmetische Grundlagen für kosmetische Desodorantien zu entwickeln, die sich durch gute Hautverträglichkeit auszeichnen.

Ferner war eine Aufgabe der vorliegenden Erfindung, Produkte auf der Basis von Mikroemulsionselastomere mit einer möglichst breiten Anwendungsvielfalt zur Verfügung zu stellen. Beispielsweise sind auf diese Weise Grundlagen für Zubereitungsformen wie Lippenpflegestifte, Antiakne-Stifte, Sonnenschutzstifte die Pforten geöffnet.

Die erfindungsgemäßen Mikroemulsionselastomere können vorteilhaft Elektrolyte, insbesondere eines oder mehrere Salze mit folgenden Anionen: Chloride, femer anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des oder der Elektrolyte sollte etwa 0,1 - 10,0 Gew.-%, besonders vorteilhaft etwa 0,3 - 8,0 Gew.% betragen, bezogen auf das Gesamtgewicht der Zubereitung.

Stellen die erfindungsgemäßen Mikroemulsionsgele Grundlagen für kosmetische Desodorantien/Antitranspirantien dar, so können alle gängigen Wirkstoffe vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Mikroemulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Famesol (3,7,11.Trimethyl-2,6,10-dodecatriën-1-ol) sowie die in den Patentoffenlegungsschriften DE-37 40 186, DE-39 38 140, DE-42 04 321, DE-42 29 707, DE-42 29 737, DE-42 37, 081, DE-43 09 372, DE-43 24 219 beschriebenen wirksamen Agenzien.

Die üblichen Antitranspiranswirkstoffe können ebenfalls vorteilhaft in den erfindungsgemäßen Mikroemulsionselastomere verwendet werden, insbesondere Adstringentien, beispielsweise basische Aluminiumchloride.

Als Treibmittel für erfindungsgemäße kosmetische, dermatologische, medizinische oder technische Elastomere sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden. Auf diese Weise können zum Mikroemulsionselastomer-Schäume hergestellt werden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Bei der Verwendung von in der Ölphase unlöslichen Treibmitteln werden mit Hohlräumen (Blasen etc) erfindungsgemäßen O/W-Mikroemulsionselastomere zugänglich. Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:

- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure-(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:

- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bomylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl) propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrunde steht wie die Bekämpfung der oxidativen Beanspruchung der Haut. Es ist aber auch günstig, die erfindungsgemäßen Mikroemulsionsgele mit einem Gehalt an einem oder mehreren Antioxidantien zu versehen, wenn die Zubereitungen einem anderen Zwecke dienen sollen, z.B. als Desodorantien oder Sonnenschutzmittel.

Besonders vorteilhaft werden die Antioxidantien gewählt aus der Gruppe bestehend aus

Aminosäuren (z.B. Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptahioninsulfoximin) in sehr geringen verträglichen Dosierungen (z.B. pmol bis μmol/kg), femer (Metall)-Chelatoren (z.B. α-Hydroxyfettsäuren, α-Hydroxypalmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 50 Gew.-%, besonders bevorzugt 0,05 - 40 Gew.-%, insbesondere 1 - 30 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 30 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 30 Gew.-%, bezogen auf das Gesamtgewicht der Formulierung, zu wählen.

Es ist dem Fachmanne natürlich bekannt, daß anspruchsvolle Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Erfindungsgemäß können Wirkstoffe auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Obgleich selbverständlich auch die Verwendung hydrophiler Wirkstoffe erfindungsgemäß begünstigt ist, ist ein weiterer Vorteil der erfindungsgemäßen Mikroemulsionselastomere, daß die hohe Anzahl feinstzerteilter Tröpfchen gerade öllösliche bzw. lipophile Wirkstoffe mit besonders großer Wirksamkeit biologisch verfügbar macht.

Vorteilhaft ist es auch, die Wirkstoffe aus der Gruppe der rückfettenden Substanzen zu wählen, beispielsweise Purcellinöl, Eucerit® und Neocerit®.

Die erfindungsgemäßen vorgesehenen Zusammensetzungen enthalten außer den vorgenannten Verbindungen Wasser und gegebenenfalls übliche Zusatzstoffe, beispielsweise Parfüm, Verdicker, Farbstoffe, antimikrobielle

Stoffe, rückfettende Agentien, Komplexierungs- und Sequestrierungsagentien, Perlglanzagentien, Pflanzenextrakte, Vitamine, Wirkstoffe, Pigmente, Filmbildner Bakterizide, Viruzide, Parfüme, Substanzen zum Verhindern des Schäumens, Farbstoffe, Pigmente, die färbende Wirkung haben, weitere, nicht unter die Definition der erfindungsgemäßen Verdicker fallende Verdickungsmittel, oberflächenaktive Substanzen, Emulgatoren, weichmachende, anfeuchtende und/oder feuchthaltende Substanzen, entzündungshemmende Substanzen, Medikamente, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel und dergleichen.

Als pharmazeutische Wirkstoffe sind erfindungsgemäß grundsätzlich alle Wirkstoffklassen geeignet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.

Insbesondere können auch vorteilhaft werden Gemische der vorstehend genannten Lösungsmittel verwendet werden.

Als weitere Bestandteile können verwendet werden Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Erfindungsgemäße Zusammensetzungen können auch, je nach Vemetzungsgrad, als technische oder medizinische Gele oder gar formstabile Filme Verwendung finden.

Sie können auch gewünschtenfalls vor oder nach der eigentlichen Vemetzungsreaktion aufgeschämt werden.

In besonderem Maße zeichnen sich die erfindungsgemäßen technischen oder medizinischen Gele, Filme oder Schäume durch eine extrem hohe Wasseraufnahmekapazität aus. Die Wasseraufnahmefähigkeit kann beispielsweise das Hunderfache des Trockeneigengewichtes der erfindungsgemäßen technischen oder medizinischen Gele, Filme oder Schäume betragen.

Die Hilfs- und Zusatzstoffe können ebenfalls aus den üblichen Substanzklassen gewählt werden. Insbesondere sind vorteilhaft Farbstoffe, Pigmente, Lichtschutzmittel, Konservierungsmittel, Duftstoffe, Antimikrobiell wirksame Substanzen, sonstige Wirkstoffe wie beispielsweise kühlend wirkende Substanzen (z.B.Menthol) oder solche die die Durchblutung fördern oder ein Wärmegefühl erzeugen und so weiter.

Besonders vorteilhaft können die erfindungsgemäßen technischen oder medizinischen Gele, Filme oder Schäume nach an sich bekannten Verfahren auf flächige Träger, z.B. Gewebe, Gewirke, Vliese, Folien oder dergleichen aufgetragen werden.

Erfindungsgemäße technische oder medizinische Filme oder formbeständige Schäume können sowohl als eher flächige Geiblde als auch als eher raumfüllende Gebilde ausgestaltet werden. Es ist möglich und gegebenenfalls vorteilhaft, sie mit einer oberflächlichen Beschichtung, beispielsweise einer Beschichtung mit Selbstklebemassen zu versehen.

Im nachfolgenden werden vorteilhafte Ausgestaltungsformen der vorliegenden Erfindung anhand einiger Beispiele vorgestellt. Alle Mengenangaben, Prozentangaben oder Teile beziehen sich, soweit nicht anders angegeben, auf Gewichts-%.

**Beispiel 1**

| | Gew.-% |
|---|---|
| Glycerylisostearat | 2,400 |
| PEG-15-Cetylisostearylalkohol | 4.800 |
| Tridecylisononanoat | 1,670 |
| Polymer 1 | 1,000 |
| Cyclomethicon | 3,300 |
| Butylenglycol | 3,000 |
| Farnesol | 0,300 |
| Glycerinmonocaprat | 0,100 |
| Parfum, Antioxidantien | q.s. |
| Wasser | ad 100,000 |

Polymer 1 : 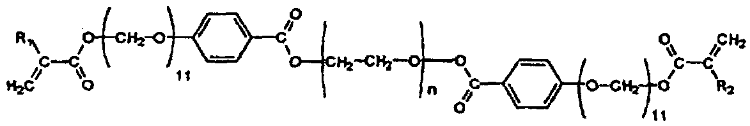 n= 800

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 2**

| | Gew.-% |
|---|---|
| Glycerylisostearat | 1,800 |
| PEG-15-Cetylstearylalkohol | 5,200 |
| Sorbitol | 2,900 |
| Isotridecylisononanoat | 3,300 |
| Polymer 2 | 1,000 |
| Cyclomethicon | 6,600 |
| Glycerinmonocaprat | 0,100 |
| Aluminiumchlorhydrat | 3,883 |
| Parfum, Antioxidantien | q.s. |
| Wasser | ad 100,000 |

Polymer 2:

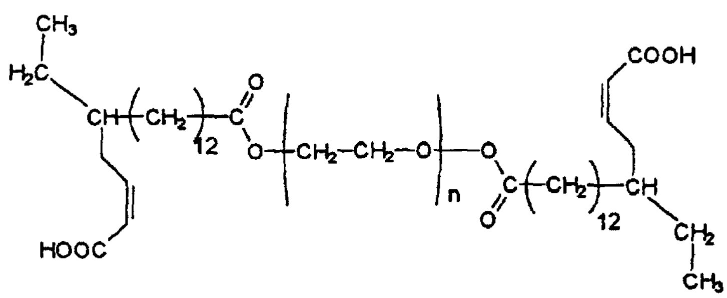

n= 400

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 3**

| | Gew.-% |
|---|---|
| Glycerylisostearat | 1,800 |
| PEG-17-Cetylstearylalkohol | 5,200 |
| Polymer 3 | 1,000 |
| Isotridecylisononanoat | 10,000 |
| Sorbitol | 2,900 |
| Glycerinmonocaprat | 0,100 |
| Antioxidantien | q.s. |
| Wasser | ad 100,000 |

**Polymer 3:**

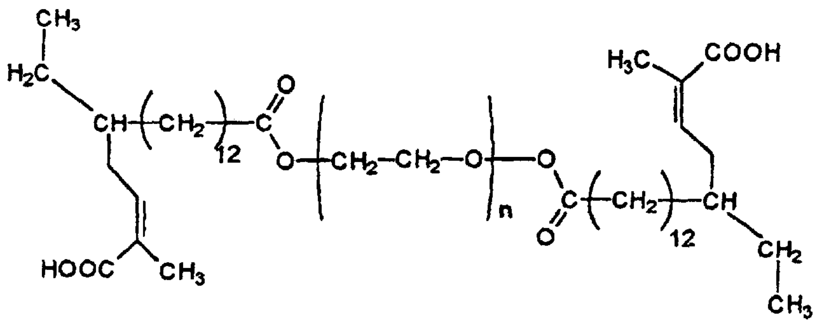

n= 400

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 4**

| Desodorierende Zubereitung | |
|---|---|
| | Gew.-% |
| Sorbitanmonoisostearat | 2,300 |
| PEG-15-Cetylstearylalkohol | 4,600 |
| Sorbitol | 2,900 |
| Cyclomethicon | 6,600 |
| Polymer 3 | 1,000 |
| Isotridecylisononanoat | 3,300 |
| Glycerinmonocaprat | 0,100 |
| | |
| Antioxidantien | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 5**

| | Gew.-% |
|---|---|
| Digycerylmonoisostearat | 1,800 |
| PEG-15-Cetylstearylalkohol | 5,100 |
| $C_{12-15}$-Alkylbenzoat | 5,000 |
| PEG-1000-Dimethacrylate | 1,000 |
| Octylisostearat | 5,000 |
| Sorbitol | 2,900 |
| Glycerinmonocaprat | 0,100 |
| Antioxidantien | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 6**

| | Gew.-% |
|---|---|
| Diglycerylmonoisostearat | 2,300 |
| PEG-15-Cetylstearylalkohol | 4,600 |
| Cyclomethicon | 6,600 |
| PEG-800-Diretinat | 1,000 |
| Sorbitol | 2,900 |
| Isotridecylisononanoat | 3,300 |
| Glycerinmonocaprat | 0,100 |
| Antioxidantien | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für -2 h. Man erhält ein erfindungsgemäßes Elastomer.

**Beispiel 7**

| | Gew.-% |
|---|---|
| Glycerylisostearat | 1,800 |
| PEG-16-Stearylalkohol | 5.100 |
| Octylisostearat | 3,300 |
| PEG-800-Dilinolenat | 1,000 |
| Cyclomethicon | 6,600 |
| Sorbitol | 2,900 |
| Glycerinmonocaprat | 0,100 |
| | |
| Antioxidantien | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 8**

| | Gew.-% |
|---|---|
| Propylenglycolmonoisostearat | 2,300 |
| PEG-15-Cetylstearylalkohol | 4,600 |
| Isotridecylisononanoat | 3,300 |
| PEG-800 Di (4-Vinylbenzoat) | 1,000 |
| Cyclomethicon | 6,600 |
| Sorbitol | 2,900 |
| Glycerinmonocaprat | 0,100 |
| | |
| Antioxidantien | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für -2 h. Man erhält ein erfindungsgemäßes Elastomer.

**Beispiel 9**

| | Gew.-% |
|---|---|
| Glycerylisostearat | 2,400 |
| Isoceteth-20 | 4,800 |

(fortgesetzt)

| | Gew.-% |
|---|---|
| Cetearylisononanoat | 1,670 |
| Polyacrylsäuredilinolenat | 1,000 |
| Eusolex® 232 | 3,000 |
| Cyclomethicon | 3,330 |
| NaOH | 0,990 |
| Glycerin | 3,000 |
| Konservierungsmittel, Farbstoffe | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 10**

| | Gew.-% |
|---|---|
| Glycerylisostearat | 2,400 |
| PEG-60-Evening Primrose Glycerides | 4,800 |
| Hydroxethylcelluloselinolenat | 4,000 |
| Isotridecylisononanoat | 3,340 |
| Cyclomethicon | 6,660 |
| Butylenglycol | 3,000 |
| Glycerinmonocaprinat | 0,100 |
| Konservierungsmittel, Farbstoffe | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 11**

| | Gew.-% |
|---|---|
| Glycerylisolaurat | 4,588 |
| Laureth-11-carboxylsäure (90 %) | 3,754 |
| Cetearylisononanoat | 1,773 |
| RetinylAlginat | 4,000 |
| Cyclomethicon | 3,441 |
| Butylenglycol | 3,128 |
| NaOH | 0,206 |

(fortgesetzt)

| | Gew.-% |
|---|---|
| Konservierungsmittel, Farbstoffe | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 12**

| | Gew.-% |
|---|---|
| PEG-20-stearat | 4,800 |
| Glycerylisostearat | 2,400 |
| Isotridecylisononanoat | 6,660 |
| PEG-800 Di (Polylactid) | 4,000 |
| Glycerinmonocaprinat | 0,100 |
| Cyclomethicon | 3,340 |
| Butylenglycol | 3,000 |
| Famesol | 0,300 |
| Konservierungsmittel, Farbstoffe | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 13**

| | Gew.-% |
|---|---|
| Sorbitanisostearat | 2,400 |
| Isotridecylisononanoat | 1,670 |
| PEG-20-Sorbitanmonostearat | 4,800 |
| PEG-800 Diarachindonat | 4,000 |
| Butylenglycol | 3,000 |
| Glycerinmonocaprinat | 0,100 |
| Cyclomethicon | 3,330 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 14**

| | Gew.-% |
|---|---|
| Isotridecylisononanoat | 1,670 |
| PEG-20-Sorbitanmonooleat | 4,800 |
| Cyclomethicon | 3,330 |
| PEG-400 Diacrylat | 4,000 |
| Butylenglycol | 3,000 |
| Glycerinmonooleat | 2,400 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 15**

| | Gew.-% |
|---|---|
| Isotridecylisononanoat | 3,311 |
| Glycerylisostearat | 1,786 |
| Oleth-15 | 5,146 |
| PEG-400 Diacrylat | 4,000 |
| Sorbitol | 2,913 |
| Glycerinmonocaprinat | 0,194 |
| Cyclomethicon | 6,621 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 16**

| | Gew.-% |
|---|---|
| Glycerylisostearat | 2,400 |
| Ceteareth-15 | 4,800 |

(fortgesetzt)

| | Gew.-% |
|---|---|
| PEG-400 Diacrylat | 4,000 |
| Caprylic/Capric/Triglycerides | 3,340 |
| Butylenglycol | 3,000 |
| Glycerinmonocaprinat | 0,100 |
| Cyclomethicon | 6,660 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 17**

| | Gew.-% |
|---|---|
| Glycerylisostearat | 2,400 |
| PEG-15-Cetylstearylalkohol | 4,800 |
| PEG-400 Diacrylat | 4,000 |
| Dicaprylylether | 5,000 |
| Butylenglycol | 3,000 |
| Glycerinmonocaprinat | 0,100 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 18**

| | Gew.-% |
|---|---|
| Diglycerinmonoisostearat | 1,840 |
| Ceteareth-15 | 5,300 |
| PEG-400 Diacrylat | 4,000 |
| Paraffinum liquidum | 5,000 |
| Sorbitol | 3,000 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 19**

| | Gew.-% |
|---|---|
| Ceteareth-15 | 5,146 |
| Octyldodecanol | 9,932 |
| PEG-400 Diacrylat | 4,000 |
| Sorbitol | 2,913 |
| Famesol | 0,097 |
| Diglycerinmonoisostearat | 1,786 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für -2 h. Man erhält ein erfindungsgemäßes Elastomer.

**Beispiel 20**

| | Gew.-% |
|---|---|
| PEG-6 Caprylsäure/Caprinsäureglyceride | 4,800 |
| Isotridecylisononanoat | 1,670 |
| PEG-400 Diacrylat | 4,000 |
| Butylenglycol | 3,000 |
| Glycerinmonocaprinat | 2,400 |
| Cyclomethicon | 3,330 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 21**

| | Gew.-% |
|---|---|
| Glycerylisostearat | 2,400 |
| Isotridecylisononanoat | 1,670 |
| Cyclomethicon | 3,330 |

(fortgesetzt)

| | Gew.-% |
|---|---|
| PEG-400 Diacrylat | 4,000 |
| Butylenglycol | 3,000 |
| PEG-20-Glycerylisostearat | 4,800 |
| Glycerinmonocaprinat | 0,100 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Beispiel 22**

| | Gew.-% |
|---|---|
| Glycerylisolaurat | 4,600 |
| Natrium-Laureth 1-4 Sulfat (25 %-ig) | 15,000 |
| PEG-400 Diacrylat | 2,000 |
| Cyclomethicon | 3,440 |
| Cetearylisononanoat | 1,770 |
| Butylenglycol | 3,125 |
| Konservierungsmittel | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für -2 h. Man erhält ein erfindungsgemäßes Elastomer.

**Beispiel 23**

| | Gew.-% |
|---|---|
| Glycerylisostearat | 2,400 |
| Ceteth-15 | 4,800 |
| Cetylpalmitat | 4,000 |
| PEG-400 Diacrylat | 4.000 |
| Butylenglycol | 3,000 |
| Parfum, Konservierungsmittel, Farbstoffe | q.s. |
| Wasser | ad 100,000 |

a) Die Bestandteile werden bei 55°C zusammengegeben und nach gleichmäßigem Verrühren über einen Zeitraum von 2-3 h auf 60°C erhitzt. Abkühlen auf RT liefert ein erfindungsgemäßes Elastomer.

b) Die Bestandteile werden bei 55°C zusammengegeben und nach gleichmäßigem Verrühren auf Raumtemperatur abgekühlt. Sodann wird mit UV-Licht (Quecksilberdampflampe, 300 Watt) bestrahlt für 1 -2 h. Man erhält ein erfindungsgemäßes Elastomer.

**Patentansprüche**

**1.** Disperse Mehrphasensysteme,

- enthaltend lediglich pharmakologisch unbedenkliche Komponenten,
- umfassend mindestens eine kontinuierliche wäßrige Phase und
- mindestens eine disperse flüssige oder feste lipoide Phase,
- wobei die einzelnen Partikel der dispersen lipoiden Phase bzw. der dispersen lipoiden Phasen durch ein makromolekulares Netzwerk miteinander verknüpft sind,
    - welches erhältlich ist durch Reaktion einer oder mehrerer Substanzen XAY,
        - deren molekulare Struktur wenigstens einen hydrophilen makromolekularen Bereich enthält, welcher mindestens zwei reaktive lipophile molekulare Bereiche verbrückt.
            - wobei diese reaktiven lipophilen molekularen Bereiche gleichartig oder unterschiedlich sein können und
            - wobei die Einzelmoleküle der Substanz oder der Substanzen XAY über diese reaktiven lipophilen molekularen Bereichen miteinander intermolekulare Reaktionen eingehen können,
            - wobei keine ungesättigten Comonomere eingesetzt werden,
        - dergestalt, daß die reaktiven, lipophilen molekularen Bereiche ins Innere der Partikel der dispersen flüssigen oder festen lipoiden Phase eintauchen und/oder an die Oberfläche der Partikel der dispersen flüssigen oder festen lipoiden Phase andocken und sodann durch Einwirkung von Wärme und/oder Licht miteinander intermolekulare Reaktionen eingehen.

**2.** Mehrphasensysteme nach Anspruch 1, **dadurch gekennzeichnet, daß** die Substanz oder Substanzen XAY, Strukturschemata wie folgt aufweisen:

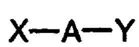
(1)

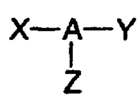
(2)

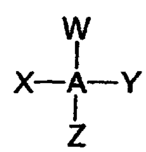
(3)

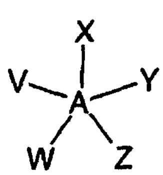
(4)

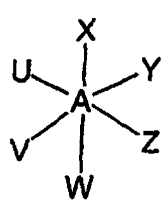
(5) ,

wobei A jeweils einen hydrophilen, die anderen Molekülbereiche miteinander verbrückenden Molekülbereich darstellt und U, V, W, X, Y und Z unabhängig voneinander gleiche oder verschiedene reaktive Molekülreste darstellen.

**3.** Mehrphasensysteme nach Anspruch 1, **dadurch gekennzeichnet, daß** wenigstens einer der Reste W, X, Y und

Z eine oder mehrere Doppel- und/oder Dreifachbindungen enthält und wenigstens einer der verbleibenden Reste geeignet ist, mit dem die Doppel- bzw. Dreifachbindungen tragenden Rest Reaktionen einzugehen.

**4.** Mehrphasensysteme nach Anspruch 1, **dadurch gekennzeichnet, daß** mindestens eine der reaktiven lipophilen molekularen Bereiche gewählt wird oder werden aus der Gruppe der Doppel- und/oder Dreifachbindungen enthaltenden Molekülreste, zum Beispiel ungesättigte Carbonsäurereste, Alkohole, Ketone, Ester, Schiffsche Basen, Aldehyde, Azoverbindungen.

**5.** Mehrphasensysteme nach Anspruch 1, **dadurch gekennzeichnet, daß** mindestens einer der reaktiven lipophilen molekularen Bereiche gewählt aus der Gruppe der Molekülreste gemäß der nachfolgenden Auflistung, in der B die Gesamtheit aus verbrückendem hydrophilem Molekülbereich und dem oder den übrigen reaktiven lipophilen molekularen Bereichen repräsentiert:

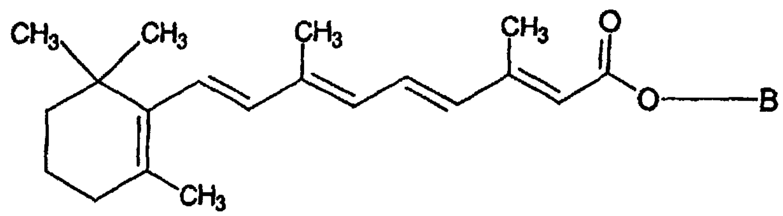

Retinsäure

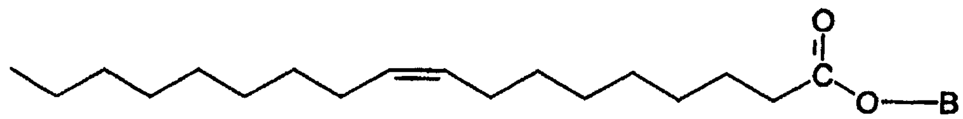

Ölsäure

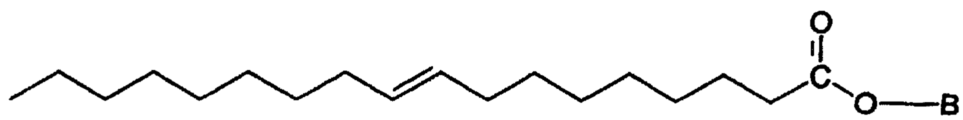

Elaidinsäure

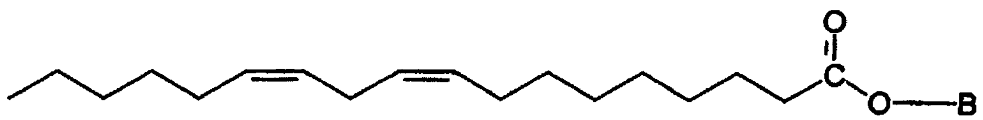

Linolsäure

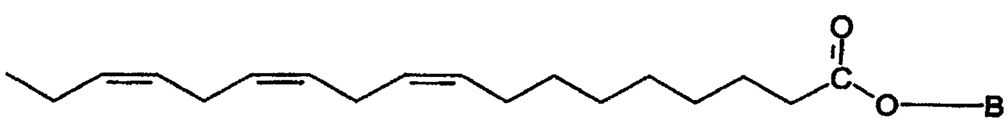

Linolensäure

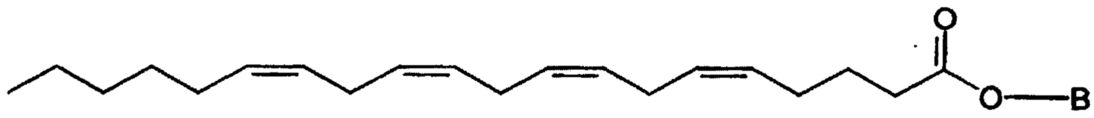

Arachidonsäure

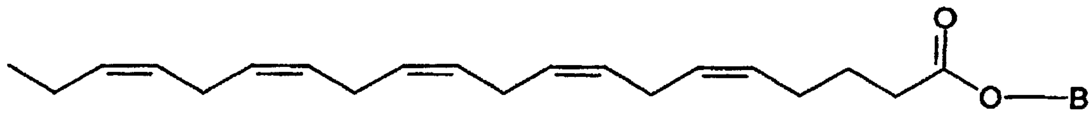

Eicosapentaënsäure

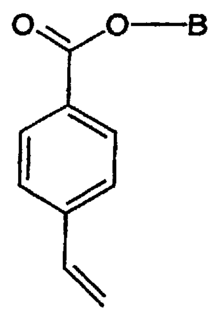

4-Vinylbenzoësäure

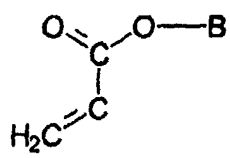

Acrylsäure

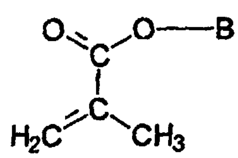

Methacrylsäure

$HC{\equiv}C{-}C{\equiv}C{-}B$

Diacetylen

$H_2C{=}CH{-}CH{=}CH{-}B$

Butadien

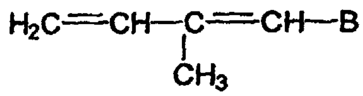

Isopren, wobei einer aber auch gewünschtenfalls mehrere bzw. alle der reaktiven lipophilen molekularen Bereiche der einzusetzenden Substanz XAY aus der Gruppe der Doppel- und/oder Dreifachbindungen enthaltenden Molekülreste, insbesondere vorteilhaft aus der Gruppe der Molekülreste gemäß der vorstehenden Auflistung gewählt werden und die reaktiven lipophilen molekularen Bereiche innerhalb eines Moleküls gleich oder verschieden sein können.

6. Mehrphasensysteme nach Anspruch 1, **dadurch gekennzeichnet, daß** der hydrophile Molekülbereich so gewählt wird, daß die Substanz XAY insgesamt wasserlöslich oder zumindest in Wasser dispergierbar ist

7. Mehrphasensysteme nach Anspruch 1, **dadurch gekennzeichnet, daß** für das Strukturschema des hydrophilen Molekülbereiches A folgende spezielleren Strukturschemata befolgt werden:

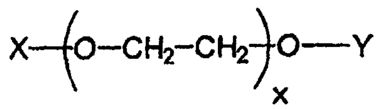

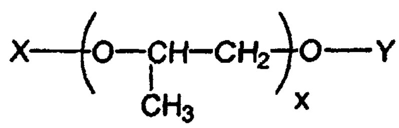

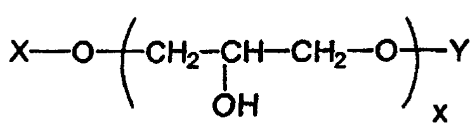

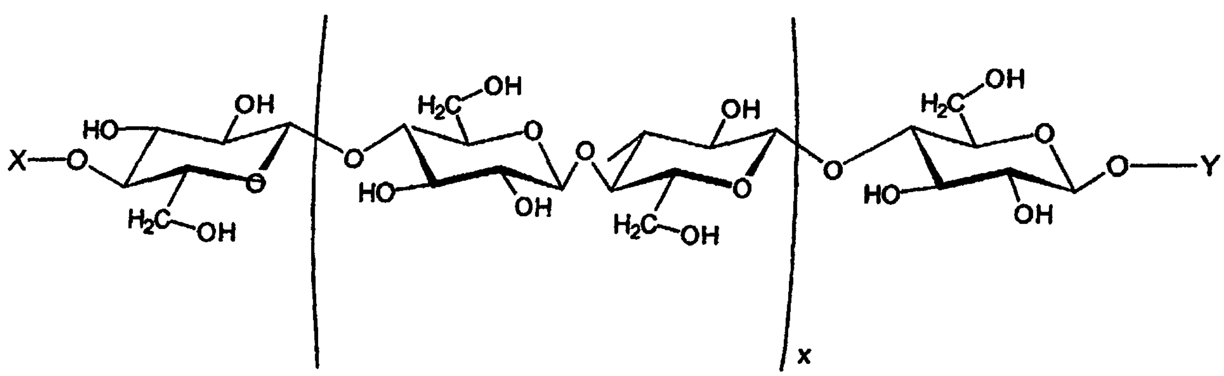

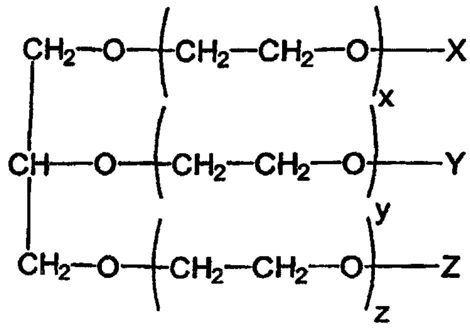

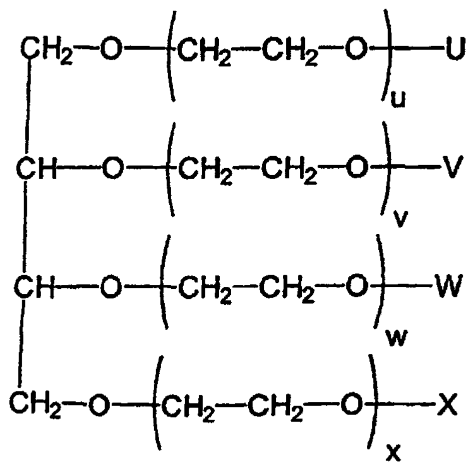

,

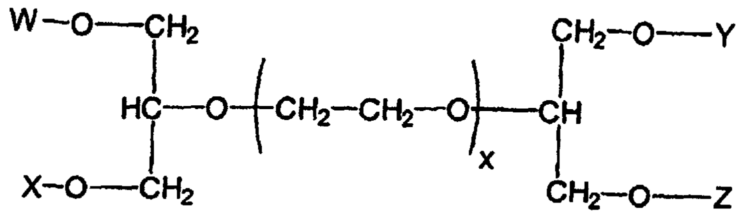

,

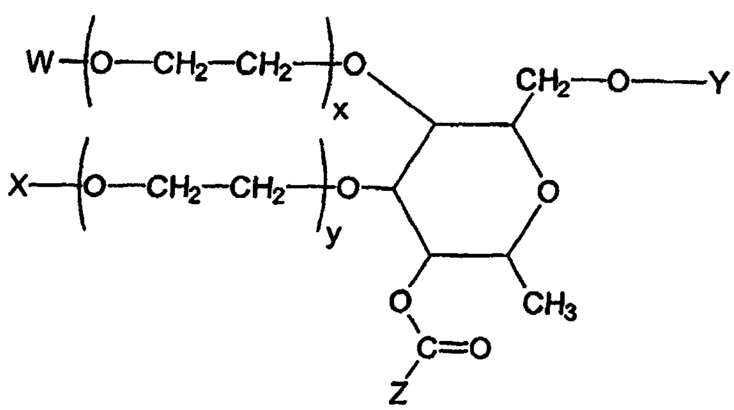

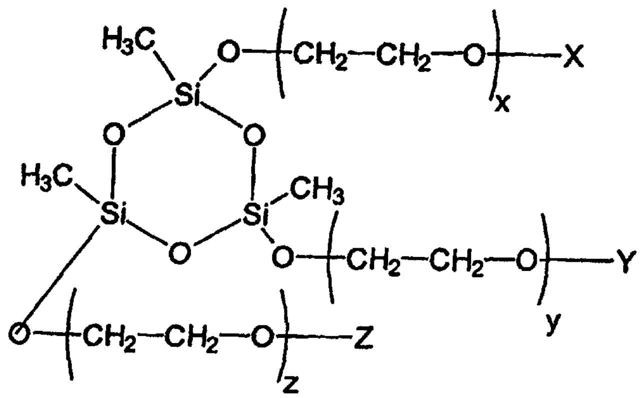

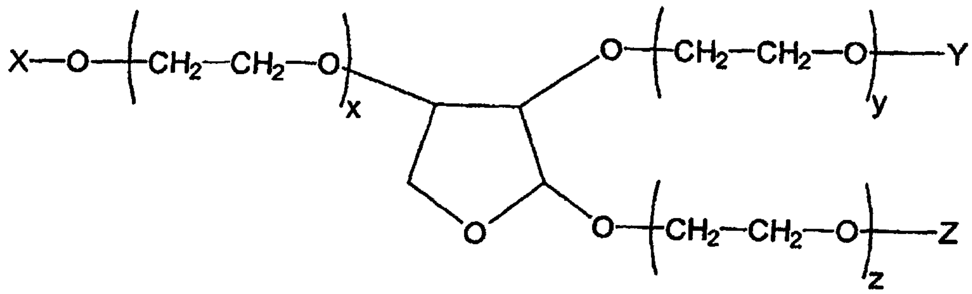

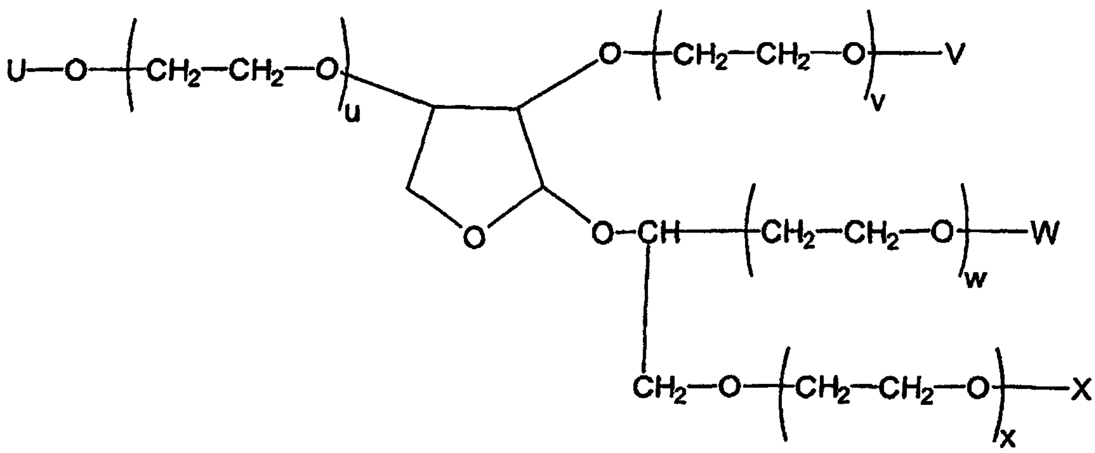

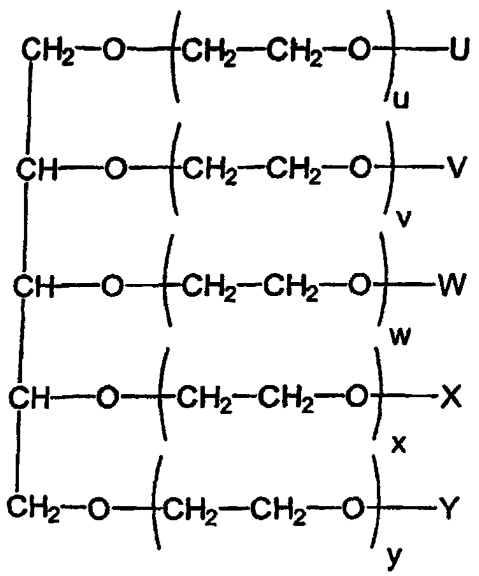

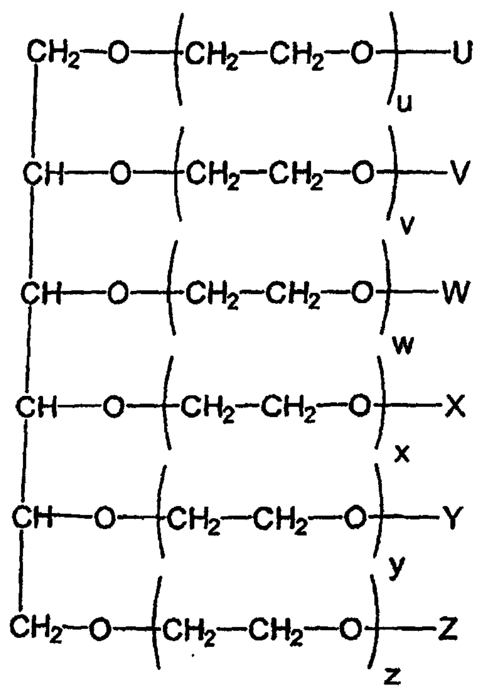

wobei U, V, W, X, Y und Z unabhängig voneinander tipophile molekulare Bereiche darstellen können, u, v, w, x, y und z dabei unabhängig voneinander Zahlen bedeuten, die es dem Gesamtmolekül erlauben, in Wasser löslich oder zumindest dispergierbar zu sein, typischerweise gewählt aus dem Bereich größer als 10, vorteilhaft aus dem Bereich 20 -100000 und wobei auch Teilsubstitution dabei möglich ist, wobei einer oder mehrere der indices u, v, w, x, y, oder z den Wert Null annehmen können und einer oder mehrere der Reste U, V, W, X, Yoder Z Wasserstoffatome darstellen können.

8. Mehrphasensysteme nach Anspruch 1, **dadurch gekennzeichnet, daß** als Substanzen des Typs XAY Dicholesterylverbindungen des Typs

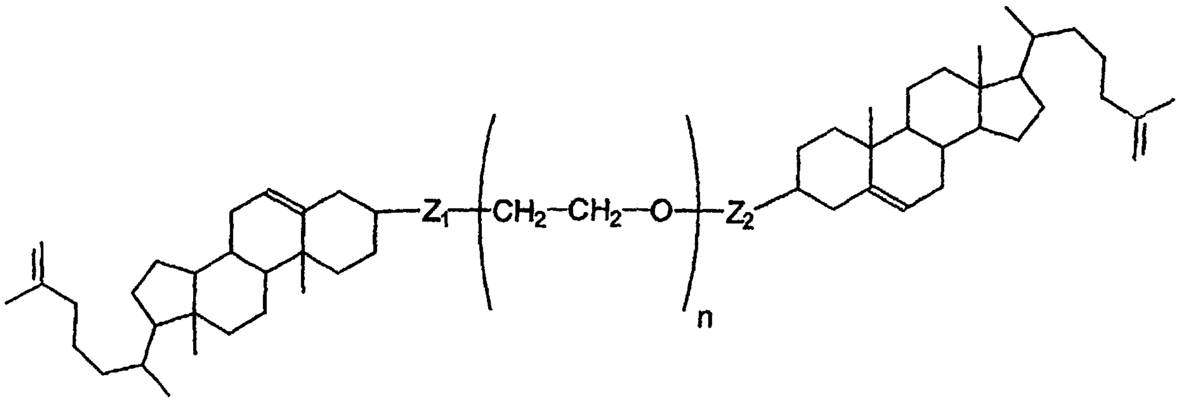

gewählt werden, wobei $Z_1$ und $Z_2$ unabhängig voneinander gewählt werden können aus der Gruppe Einfachbindung, Estergruppe, Kohlensäureestergruppe, Sauerstoff, Säureamidgruppe, Säureimidgruppe, Thiocarbonsäureestergruppe, Urethan- bzw. Carbamatgruppe.

**9.** Mehrphasensysteme nach Anspruch 8, **dadurch gekennzeichnet, daß** als Substanzen des Typs XAY Dicholesterylverbindungen des Typs

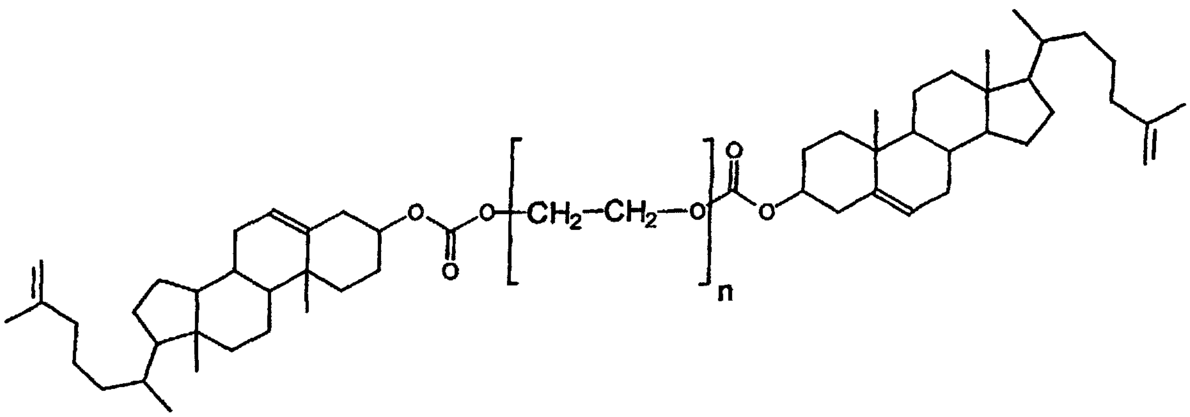

gewählt werden, wobei n Zahlen bedeutet, die es dem Gesamtmolekül erlaubt, in Wasser löslich oder zumindest dispergierbar zu sein, typischerweise gewählt aus dem Bereich größer als 10, vorteilhaft aus dem Bereich 20 bis $10^7$, ganz besonders vorteilhaft aus dem Bereich 120 bis 800.

**10.** Mehrphasensysteme nach Anspruch 1, **dadurch gekennzeichnet, daß** als Substanzen des Typs XAY Polyethylenglycol-Diacrylate sowie Polyethylenglycol-Dimethacrylate des Typs

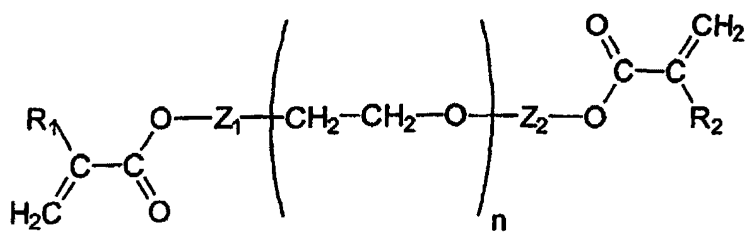

,

verwendet werden, wobei n Zahlen bedeutet, die es dem Gesamtmolekül erlaubt, in Wasser löslich oder zumindest dispergierbar zu sein, typischerweise gewählt aus dem Bereich größer als 10, vorteilhaft aus dem Bereich 20 bis $10^7$, ganz besonders vorteilhaft aus dem Bereich 120 bis 800, und wobei $Z_1$ und $Z_2$ unabhängig voneinander gewählt werden können aus der Gruppe Einfachbindung, Estergruppe, Kohlensäureestergruppe, Sauerstoff, Säureamidgruppe, Säureimidgruppe, Thiocarbonsäureestergruppe, Urethan- bzw. Carbamatgruppe. $R_1$ und $R_2$ be-

deuten unabhängig voneinander H oder Methyl,

**11.** Mehrphasensysteme nach Anspruch 10, **dadurch gekennzeichnet, daß** $Z_1$ und $Z_2$ gewählt wird aus den organischen Gruppen

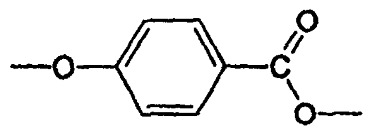

gegeben ist, wobei n Zahlen bedeutet, die es dem Gesamtmolekül erlaubt, in Wasser löslich oder zumindest dispergierbar zu sein, typischerweise gewählt aus dem Bereich größer als 10, vorteilhaft aus dem Bereich 20 bis $10^7$, ganz besonders vorteilhaft aus dem Bereich 120 bis 800.

**12.** Mehrphasensysteme nach Anspruch 1, **dadurch gekennzeichnet, daß** die Menge der Verdicker im Bereich von 0,3 bis 50 Gew.-%, insbesondere 0,5 bis 30 Gew.-% liegen, jeweils bezogen auf das Gesamtgewicht des Mikroemulsionselastomers, gewählt wird.

**Claims**

**1.** Disperse multiphase systems

- comprising only pharmacologically acceptable components,
- comprising at least one continuous aqueous phase and
- at least one disperse liquid or solid lipoidal phase,
  - where the individual particles of the disperse lipoidal phase or of the disperse lipoidal phases are linked together by a macromolecular network,
    - which is obtainable by reaction of one or more substances XAY,
      - whose molecular structure contains at least one hydrophilic macromolecular region which bridges at least two reactive lipophilic molecular regions,
      - where these reactive lipophilic molecular regions may be of the same type or different and
      - where the individual molecules of the substance or of the substances XAY can, by means of these reactive lipophilic molecular regions, undergo intermolecular reactions with one another,
      - where no unsaturated comonomers are used,
        - such that the reactive, lipophilic molecular regions dip into the inside of the particles of the disperse liquid or solid lipoidal phase and/or link to the surface of the particles of the disperse liquid or solid lipoidal phase and then undergo intermolecular reactions with one another as a result of the action of heat and/or light.

**2.** Multiphase systems according to Claim 1, **characterized in that** the substance or substances XAY have structural schemes as follows:

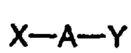
(1)

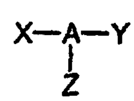
(2)

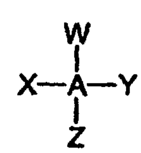
(3)

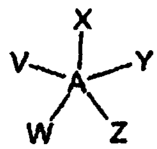

(4)

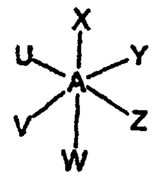

(5)

where A in each case represents a hydrophilic molecular region which bridges the other molecular regions to one another, and U, V, W, X, Y and Z, independently of one another, are identical or different reactive molecular radicals.

3. Multiphase systems according to Claim 1, **characterized in that** at least one of the radicals W, X, Y and Z contains one or more double and/or triple bonds and at least one of the remaining radicals is suitable for undergoing reactions with the radical carrying the double or triple bonds.

4. Multiphase systems according to Claim 1, **characterized in that** at least one of the reactive lipophilic molecular regions is or are chosen from the group of molecular radicals containing double and/or triple bonds, for example unsaturated carboxylic acid radicals, alcohols, ketones, esters, Schiff's bases, aldehydes, azo compounds.

5. Multiphase systems according to Claim 1, **characterized in that** at least one of the reactive lipophilic molecular regions is chosen from the group of molecular radicals according to the listing below in which B represents the entirety of bridging hydrophilic molecular region and the other reactive lipophilic molecular region(s):

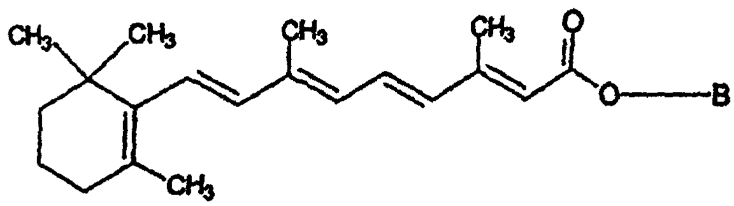

Retinoic acid

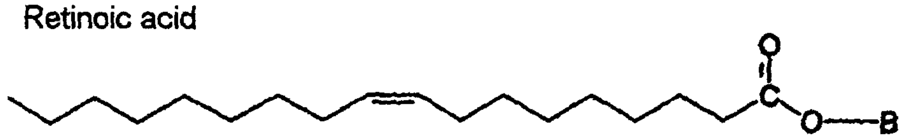

Oleic acid

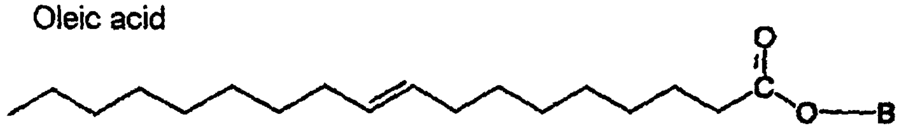

Elaidic acid

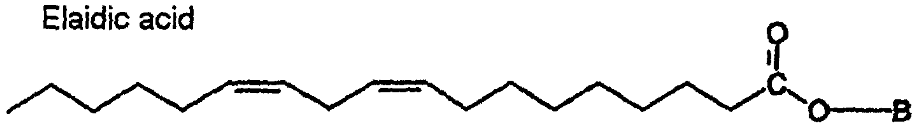

Linoleic acid

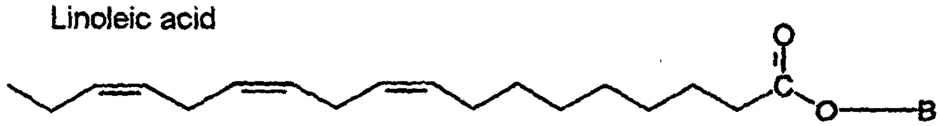

Linolenic acid

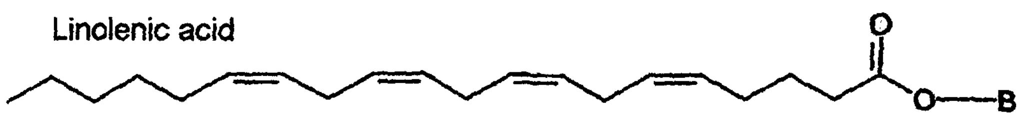

Arachidonic acid

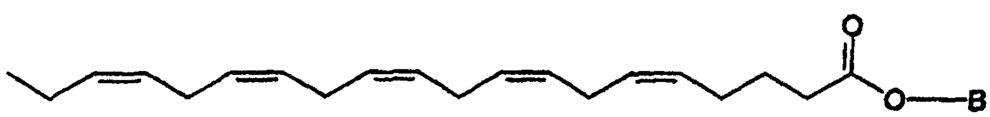

Eicosapentaenoic acid

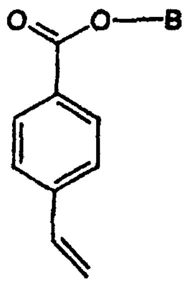

4-Vinylbenzoic acid

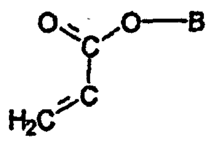

Acrylic acid

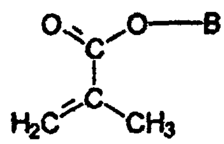

Methacrylic acid

$$HC \equiv C - C \equiv C - B$$

Diacetylene

$$H_2C=CH-CH=CH-B$$

Butadiene

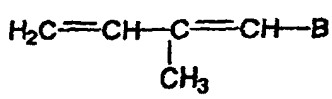

Isoprene,

where one, or else if desired two or more or all, of the reactive lipophilic molecular regions of the substance XAY to be used are chosen from the group of molecular radicals containing double and/or triple bonds, in particular advantageously from the group of molecular radicals according to the above listing, and the reactive lipophilic molecular regions within one molecule may be identical or different.

**6.** Multiphase systems according to Claim 1, **characterized in that** the hydrophilic molecular region is chosen such that the substance XAY is overall water-soluble or at least dispersible in water.

**7.** Multiphase systems according to Claim 1, **characterized in that** for the structural scheme of the hydrophilic molecular region A, the following more specific structural schemes are observed:

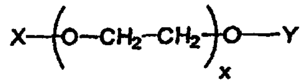

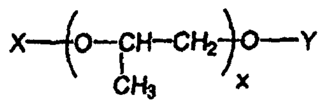

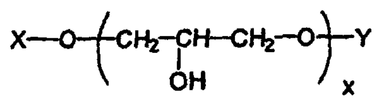

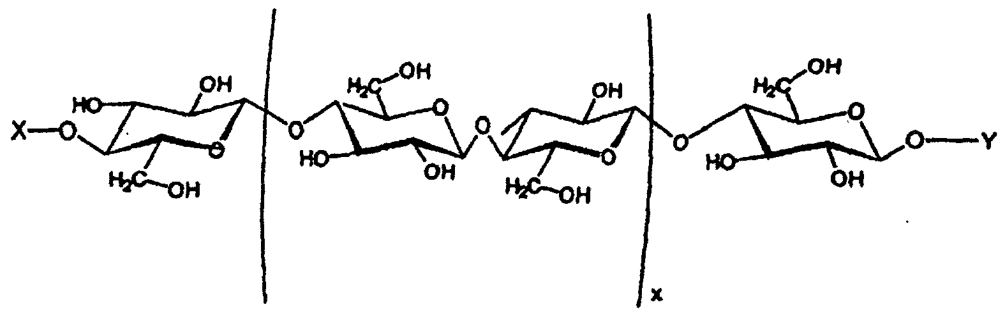

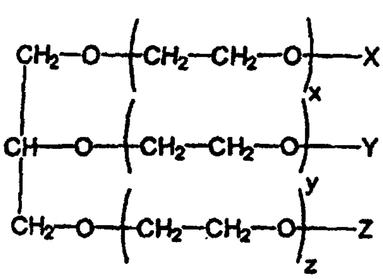

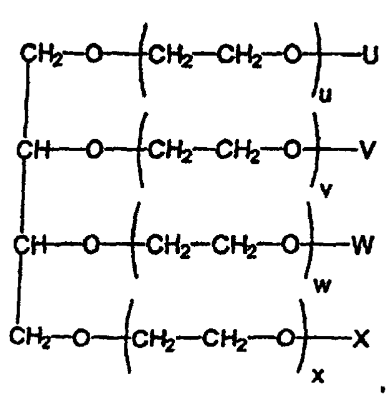

,

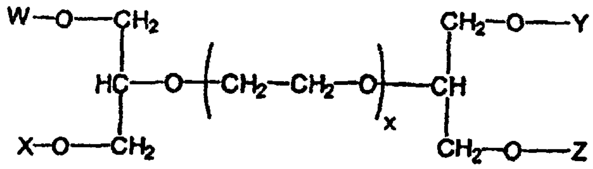

,

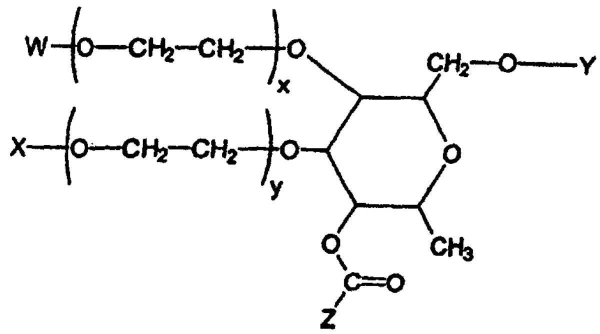

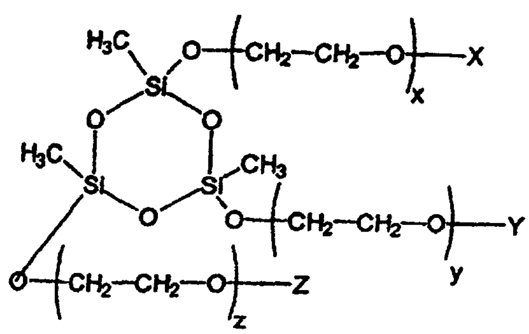

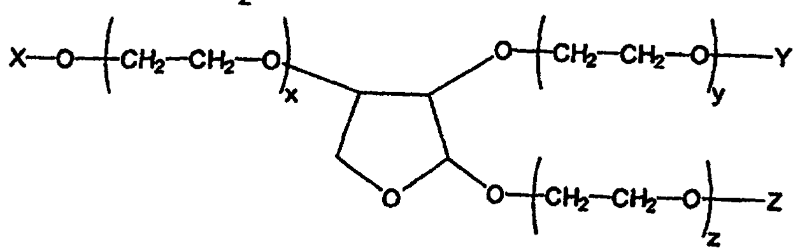

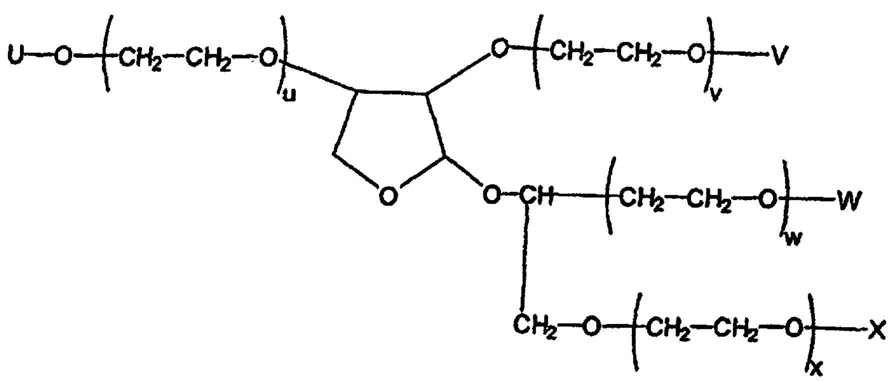

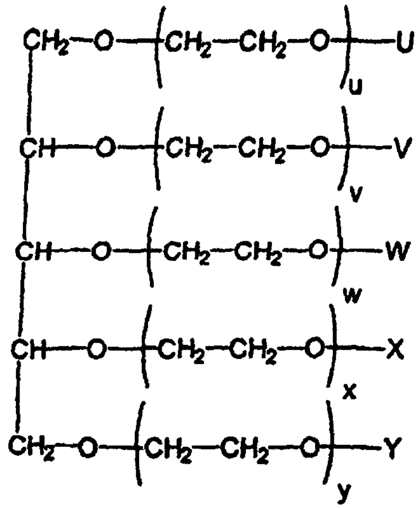

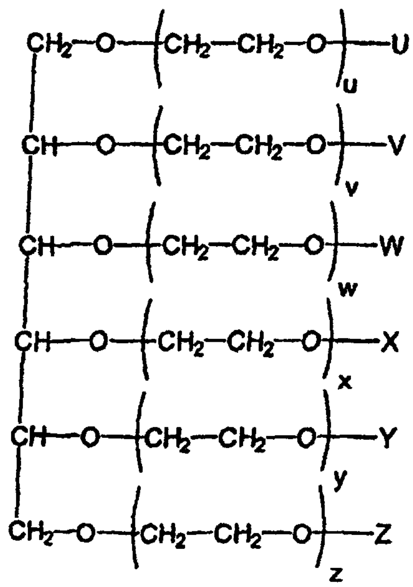

where U, V, W, X, Y and Z, independently of one another, may represent lipophilic molecular regions, u, v, w, x, y and z are here, independently of one another, numbers which allow the overall molecule to be soluble or at least dispersible in water, typically chosen from the range greater than 10, advantageously from the range 20-100,000 and where partial substitution is also possible, where one or more of the indices u, v, w, x, y, or z can assume the value zero and one or more of the radicals U, V, W, X, Y or Z can represent hydrogen atoms.

**8.** Multiphase systems according to Claim 1, **characterized in that** the substances of the type XAY chosen are dicholesteryl compounds of the type

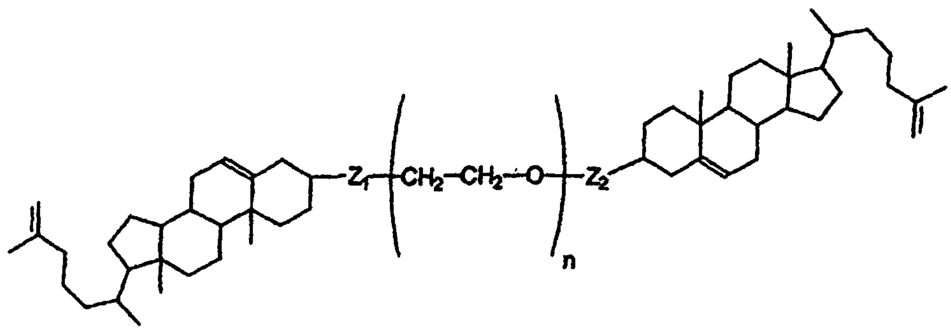

where $Z_1$ and $Z_2$, independently of one another, may be chosen from the group single bond, ester group, carbonic ester group, oxygen, acid amide group, acid imide group, thiocarboxylic ester group, urethane or carbamate group.

**9.** Multiphase systems according to Claim 8, **characterized in that** substances of the type XAY chosen are dicholesteryl compounds of the type

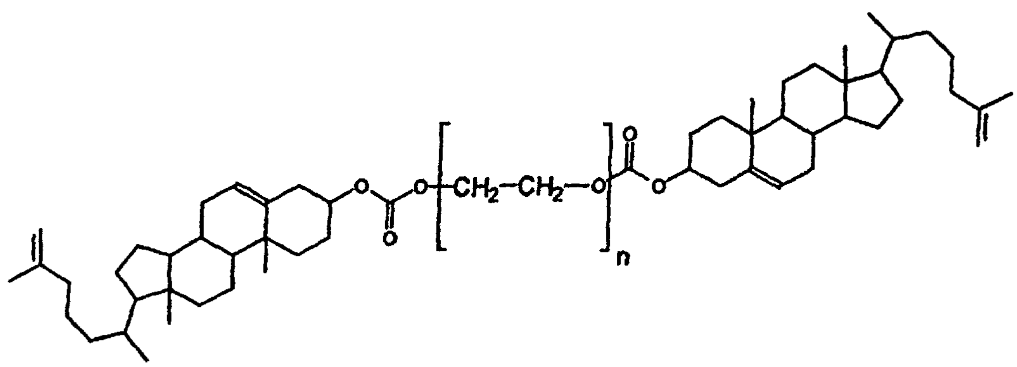

where n is a number which allows the overall molecule to be soluble or at least dispersible in water, typically chosen from the range greater than 10, advantageously from the range 20 to $10^7$, very particularly advantageously from the range 120 to 800.

**10.** Multiphase systems according to Claim 1, **characterized in that** the substances of the type XAY used are polyethylene glycol diacrylates, and polyethyleneglycol dimethacrylates of the type

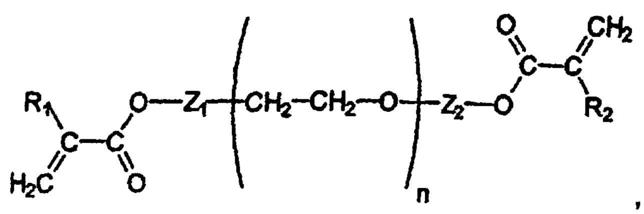

,

where n is a number which allows the overall molecule to be soluble or at least dispersible in water, typically chosen from the range greater than 10, advantageously from the range 20 to $10^7$, very particularly advantageously from the range 120 to 800, and where $Z_1$ and $Z_2$, independently of one another, can be chosen from the group consisting of single bond, ester group, carbonic ester group, oxygen, acid amide group, acid imide group, thiocarboxylic ester group, urethane or carbamate group. $R_1$ and $R_2$, independently of one another, are H or methyl,

**11.** Multiphase systems according to Claim 10, **characterized in that** $Z_1$ and $Z_2$ is chosen from the organic groups

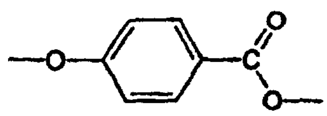

is given [sic], where n is a number which allows the overall molecule to be soluble or at least dispersible in water, typically chosen from the range greater than 10, advantageously from the range 20 to $10^7$, very particularly advantageously from the range 120 to 800.

**12.** Multiphase systems according to Claim 1, **characterized in that** the amount of the thickeners is chosen to lie in the range from 0.3 to 50% by weight, in particular 0.5 to 30% by weight, in each case based on the total weight of the microemulsion elastomer.

**Revendications**

**1.** Systèmes multiphasiques dispersés

- contenant seulement des composants pharmacologiquement acceptables,
- comprenant au moins une phase aqueuse continue et
- au moins une phase lipoïde solide ou liquide dispersée,
  - les particules individuelles de la phase lipoïde dispersée ou des phases lipoïdes dispersées étant reliées les unes aux autres par un réseau macromoléculaire,
    - qui peut être obtenu par la réaction d'une ou plusieurs substances XAY,
      - dont la structure moléculaire comporte au moins un domaine macromoléculaire hydrophile qui ponte au moins deux régions moléculaires lipophiles réactives,
        - ces régions moléculaires lipophiles réactives pouvant être identiques ou différentes et
        - les molécules individuelles de la substance ou des substances XAY pouvant participer à des réactions intermoléculaires entre elles, par ces régions moléculaires lipophiles réactives,
        - aucun comonomère insaturé n'étant utilisé,
      - de sorte que les régions moléculaires lipophiles réactives pénètrent à l'intérieur des particules de la phase lipoïde solide ou liquide dispersée et/ou se fixent à la surface des particules de la phase lipoïde solide ou liquide dispersée et ainsi entrent dans des réactions intermoléculaires entre elles, sous l'effet de la chaleur et/ou de la lumière.

**2.** Systèmes multiphasiques selon la revendication 1, **caractérisés en ce que** la substance ou les substances XAY présentent les schémas de structure comme suit :

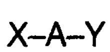

(1)

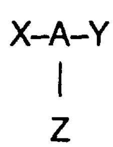

(2)

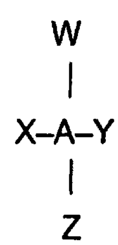

(3)

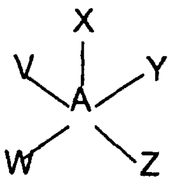

(4)

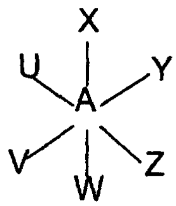

(5)

A représentant chaque fois une région moléculaire hydrophile pontant entre elles les autres régions moléculaires et U, V, W, X, Y et Z représentant, indépendamment les uns des autres, des restes moléculaires réactifs identiques ou différents.

**3.** Systèmes multiphasiques selon la revendication 1, **caractérisés en ce qu'**au moins l'un des radicaux W, X, Y et Z comporte une ou plusieurs doubles et/ou triples liaisons et au moins l'un des autres radicaux est approprié à entrer en des réactions avec le radical portant les doubles ou triples liaisons.

**4.** Systèmes multiphasiques selon la revendication 1, **caractérisés en ce qu'**au moins une ou plusieurs des régions moléculaires lipophiles réactives est ou sont choisies dans le groupe des restes moléculaires comportant des doubles et/ou des triples liaisons, par exemple, des restes d'acides carboxyliques insaturés, des alcools, des cétones, des esters, des bases de Schiff, des aldéhydes, des composés azo.

**5.** Systèmes multiphasiques selon la revendication 1, **caractérisés en ce qu'**au moins l'une des régions moléculaires lipophiles réactives est choisie dans le groupe des restes moléculaires selon la liste suivante, dans laquelle B représente l'ensemble de la région moléculaire hydrophile pontant et de l'autre ou des autres régions moléculaires lipophiles réactives, sont particulièrement avantageux :

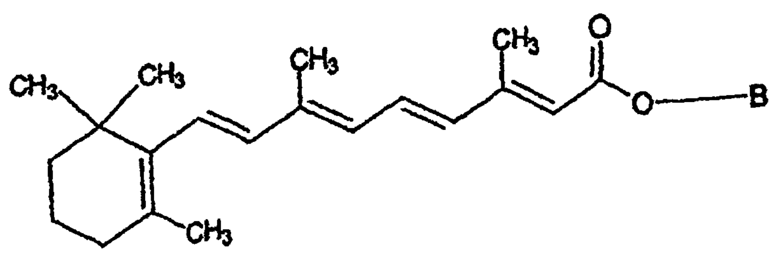

Acide rétinique

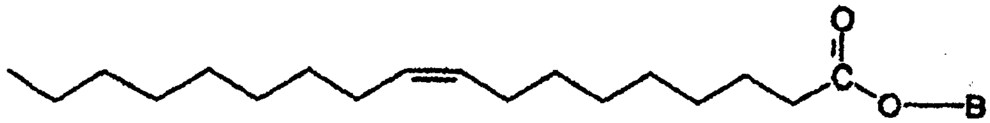

Acide oléique

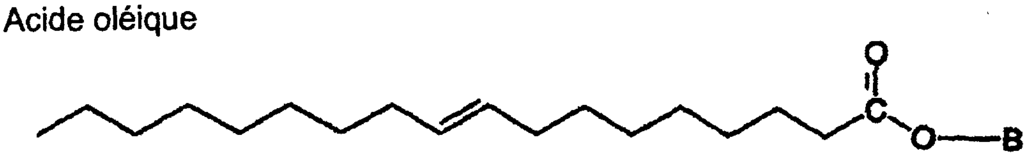

Acide élaïdique

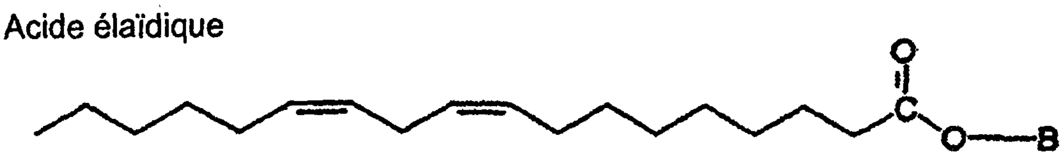

Acide linoléique

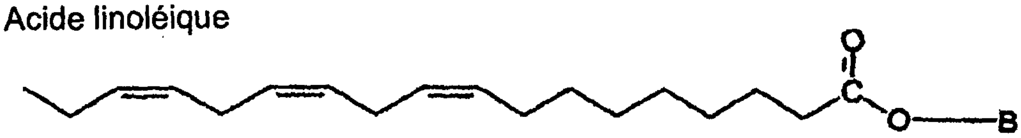

Acide linolénique

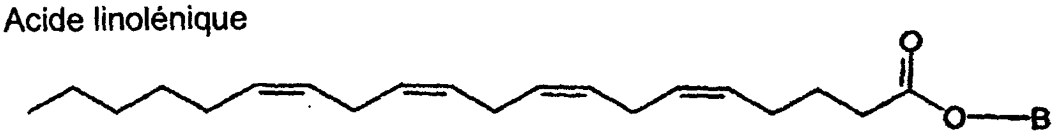

Acide arachidonique

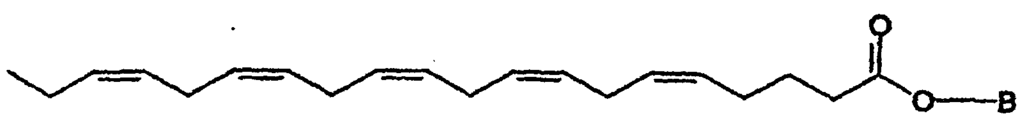

Acide eicosapentaénoïque

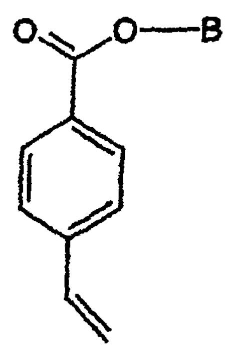

Acide 4-vinylbenzoïque

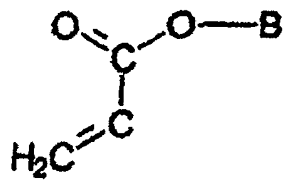

Acide acrylique

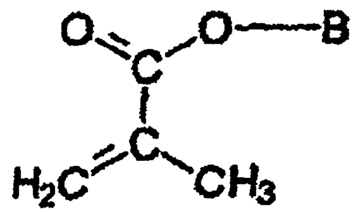

Acide méthacrylique

$HC \equiv C\text{-}C \equiv C\text{-}B$

Diacétylène

$H_2C\text{=}CH\text{-}CH\text{=}CH\text{-}B$

Butadiène

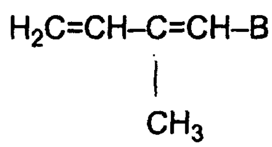

Isoprène,

un ou bien, si on le désire, plusieurs ou la totalité des régions moléculaires lipophiles réactives de la substance XAY à utiliser étant choisie(s) dans le groupe des restes moléculaires contenant des doubles et/ou triples liaisons, de façon particulièrement avantageuse, dans le groupe des restes moléculaires selon la liste précédente et les régions moléculaires lipophiles réactives à l'intérieur d'une molécule pouvant être identiques ou différentes.

**6.** Systèmes multiphasiques selon la revendication 1, **caractérisés en ce que** la région moléculaire est choisie de telle façon que la substance XAY est dans son ensemble hydrosoluble ou au moins dispersable dans l'eau.

**7.** Systèmes multiphasiques selon la revendication 1, **caractérisés en ce que** pour le schéma de structure de la région moléculaire hydrophile A on suit les schémas de structure plus particuliers suivants :

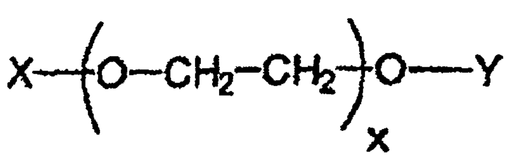

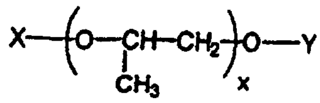

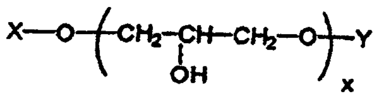

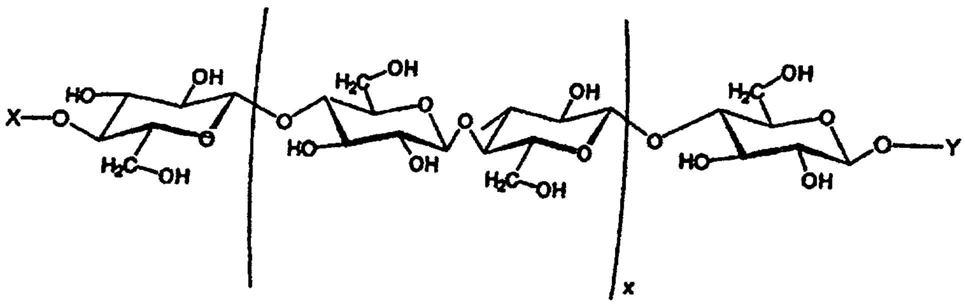

,

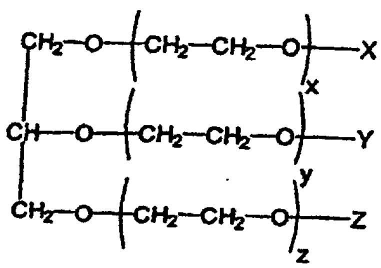

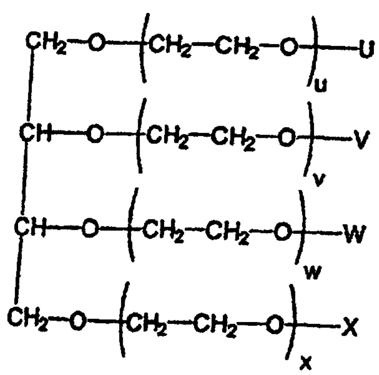

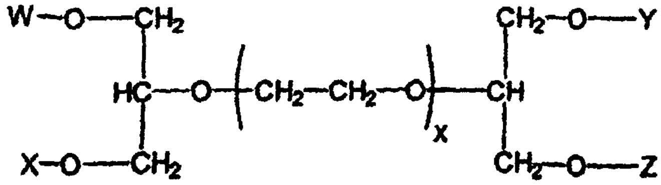

,

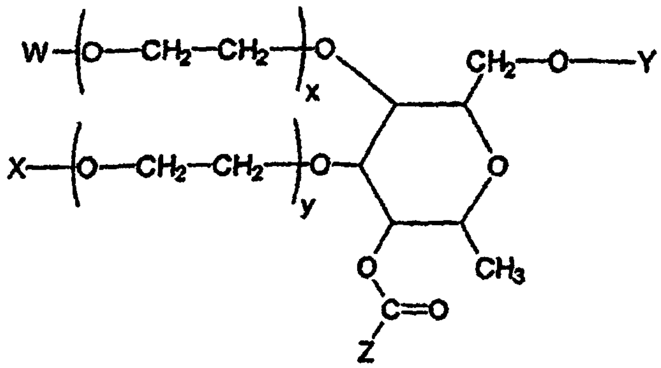

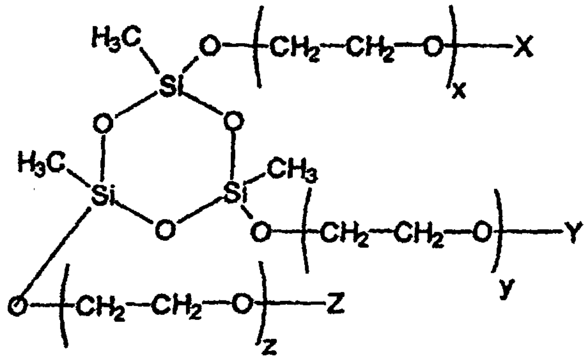

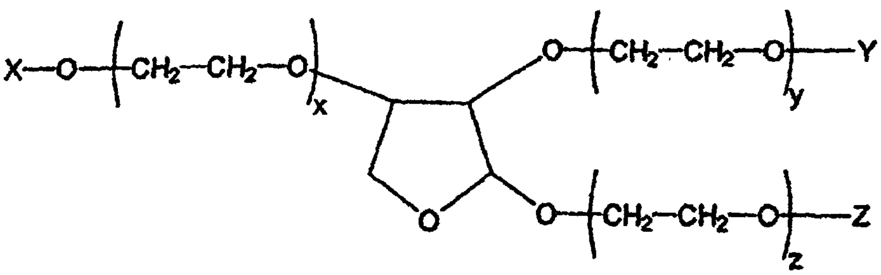

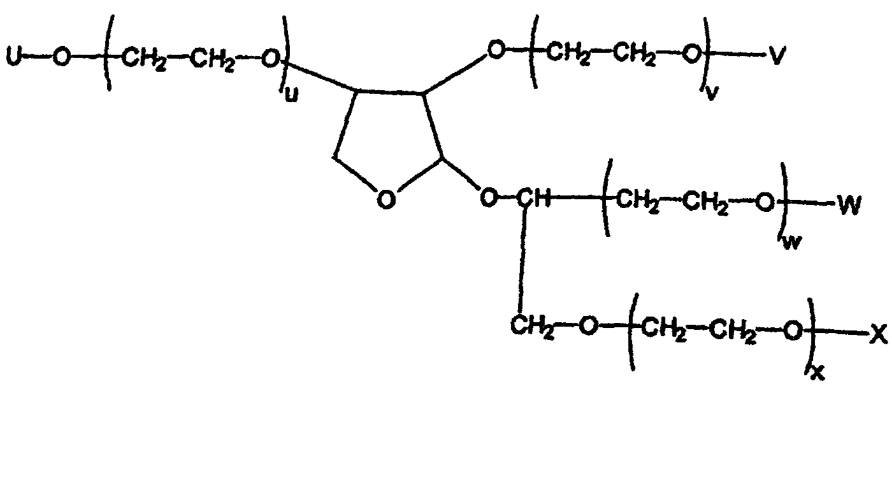

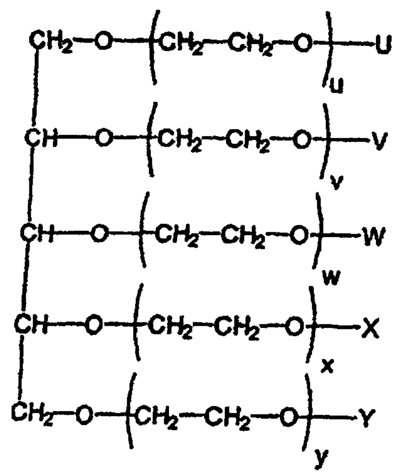

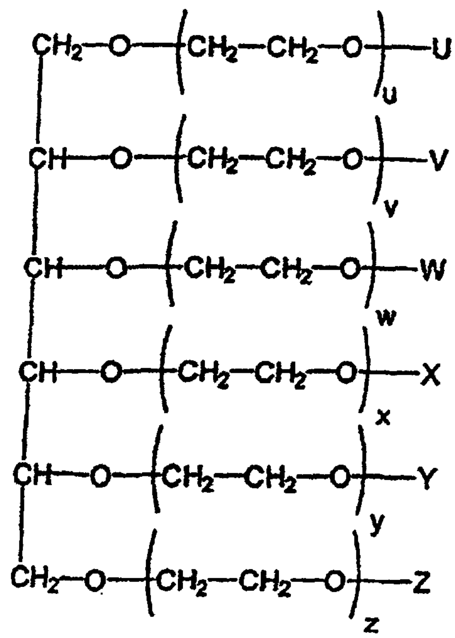

U, V, W, X, Y et Z pouvant représenter, indépendamment les uns des autres, des régions moléculaires lipophiles, u, v, w, x, y et z représentant, indépendamment les uns des autres, des nombres permettant à la molécule dans son ensemble d'être soluble ou au moins dispersable dans l'eau, normalement choisis dans la plage de plus de 10, avantageusement dans la plage de 20 - 100 000 et une substitution partielle étant en outre possible, un ou plusieurs des indices u, v, w, x, y et z pouvant adopter la valeur zéro et un ou plusieurs des radicaux U, V, W, X, Y et Z pouvant représenter des atomes d'hydrogène.

**8.** Systèmes multiphasiques selon la revendication 1, **caractérisés en ce qu'**on choisit en tant que substances du type XAY des composés dicholestéryle du type

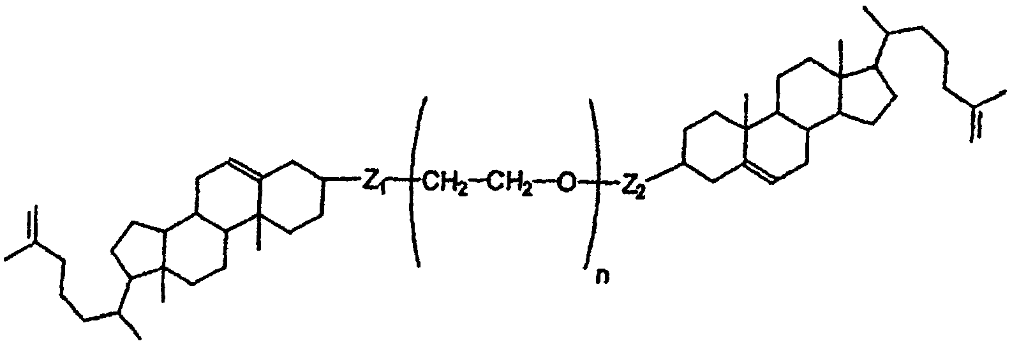

dans lesquels $Z_1$ et $Z_2$ peuvent être choisis, indépendamment l'un de l'autre, dans le groupe constitué par une simple liaison, un groupe ester, un groupe ester d'acide carbonique, un atome d'oxygène, un groupe amido, un groupe imido, un groupe ester d'acide thiocarboxylique, le groupe uréthanne ou carbamate.

**9.** Systèmes multiphasiques selon la revendication 8, **caractérisés en ce qu'**on choisit en tant que substances du type XAY des composés dicholestéryle du type

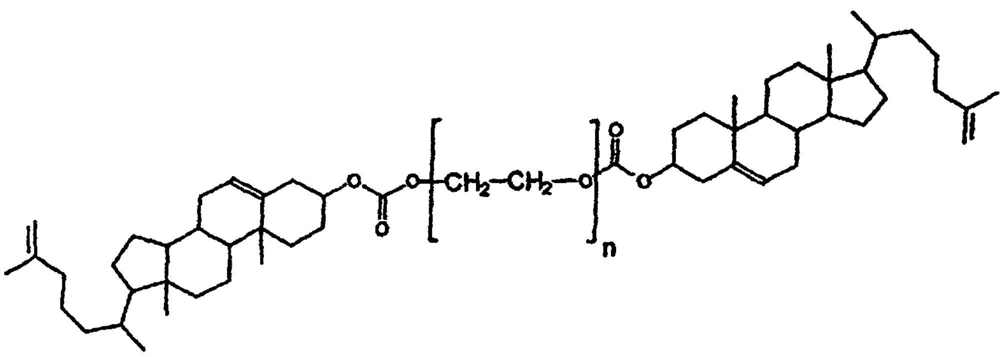

n représentant des nombres permettant à la molécule dans son ensemble d'être soluble ou au moins dispersable dans l'eau, normalement choisis dans la plage supérieure à 10, avantageusement dans la plage allant de 20 à $10^7$, de façon tout particulièrement avantageuse, dans la plage allant de 120 à 800.

**10.** Systèmes multiphasiques selon la revendication 1, **caractérisés en ce qu'**on utilise comme substances de type XAY des diacrylates de polyéthylèneglycol ainsi que des diméthacrylates de polyéthylèneglycol du type

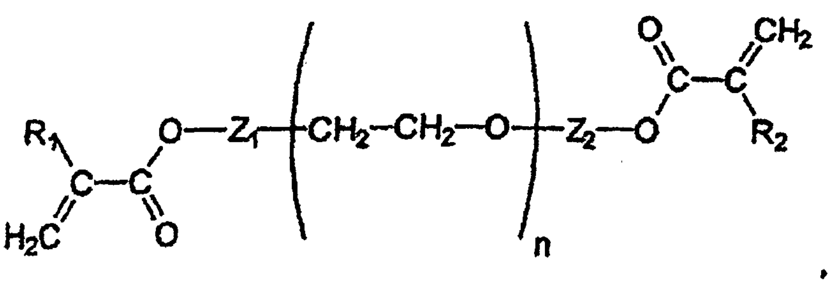

n représentant des nombres qui permettent à la molécule dans son ensemble d'être soluble ou au moins dispersable dans l'eau, normalement choisis dans la plage de plus de 10, avantageusement dans la plage allant de 20 à $10^7$, de façon tout particulièrement avantageuse dans la plage allant de 120 à 800, et $Z_1$ et $Z_2$ pouvant être choisis, indépendamment l'un de l'autre, dans le groupe constitué par une liaison simple, un groupe ester, un groupe ester d'acide carbonique, un atome d'oxygène, un groupe amido, un groupe imido, un groupe ester d'acide thiocarboxylique, le groupe uréthanne ou carbamate. $R_1$ et $R_2$ représentent, indépendamment l'un de l'autre, H

ou le groupe méthyle.

**11.** Systèmes multiphasiques selon la revendication 10, **caractérisés en ce que** $Z_1$ et $Z_2$ sont choisis parmi les groupes organiques

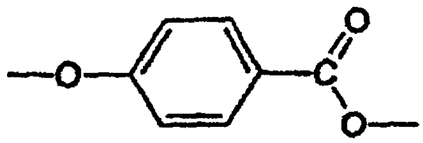

dans laquelle n représente des nombres permettant à la molécule dans son ensemble d'être soluble ou au moins dispersable dans l'eau, normalement choisis dans la plage de plus de 10, avantageusement dans la plage allant de 20 à $10^7$, de façon tout particulièrement avantageuse dans la plage allant de 120 à 800.

**12.** Systèmes multiphasiques selon la revendication 1, **caractérisés en ce que** la quantité des épaississants est choisie dans la plage allant de 0,3 à 50 % en poids, en particulier de 0,5 à 30 % en poids, dans chaque cas par rapport au poids total de l'élastomère en microémulsion.

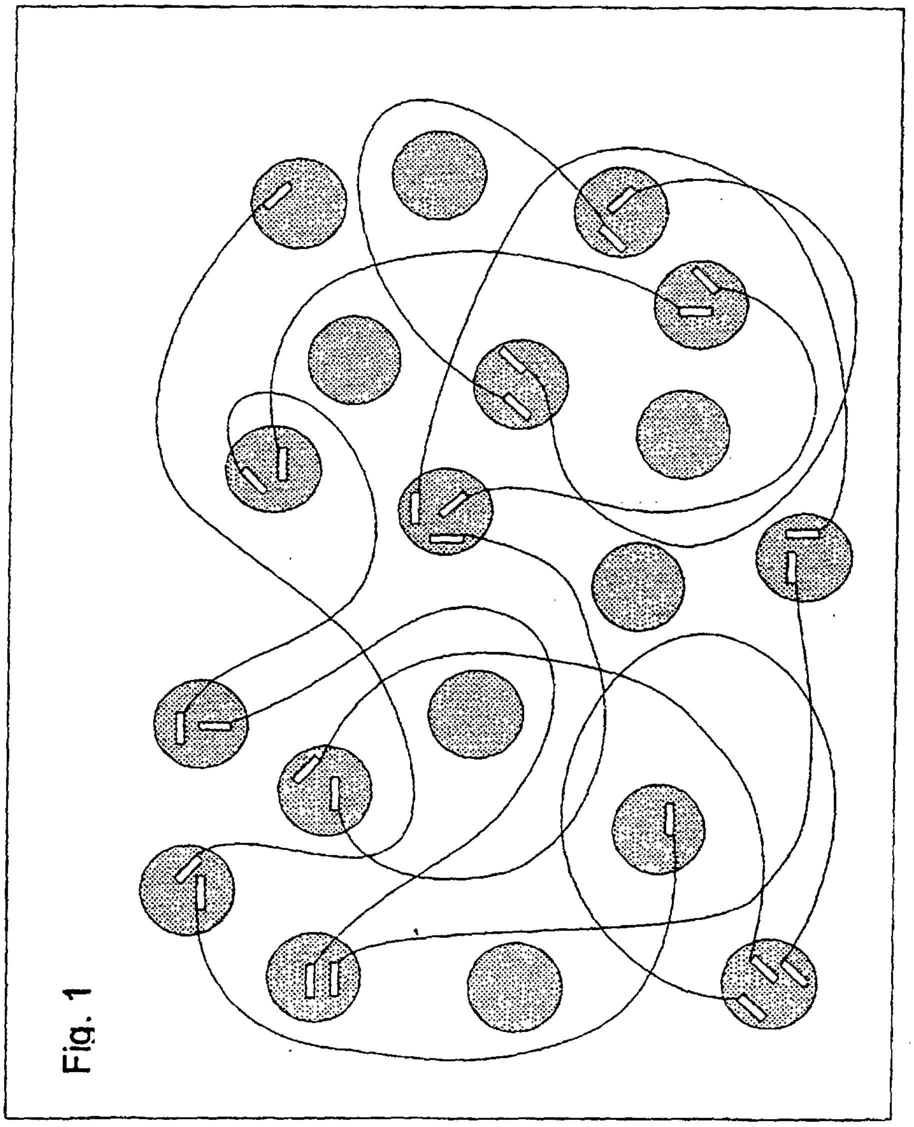

Fig. 1

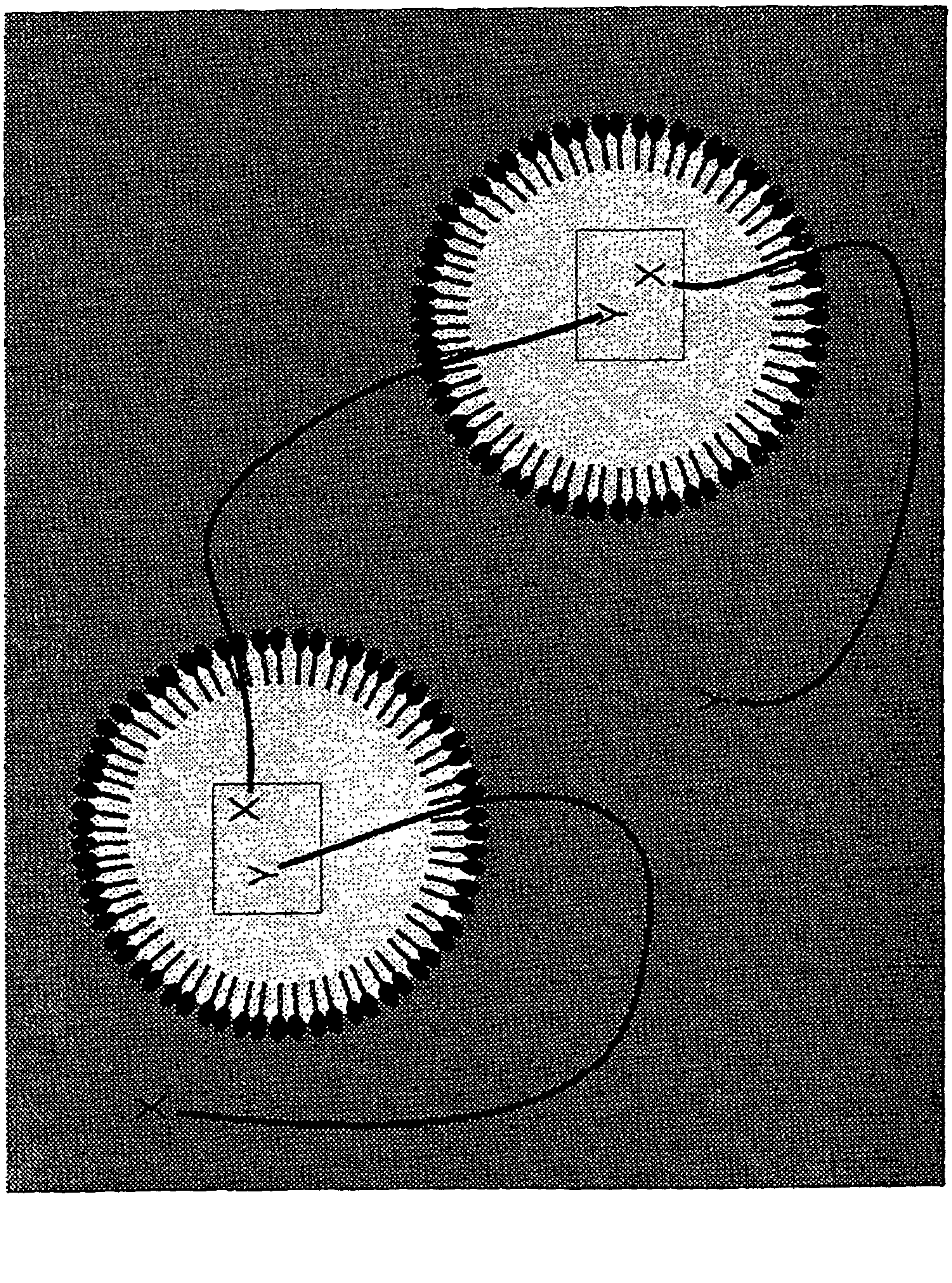

Fig. 2

Fig. 3

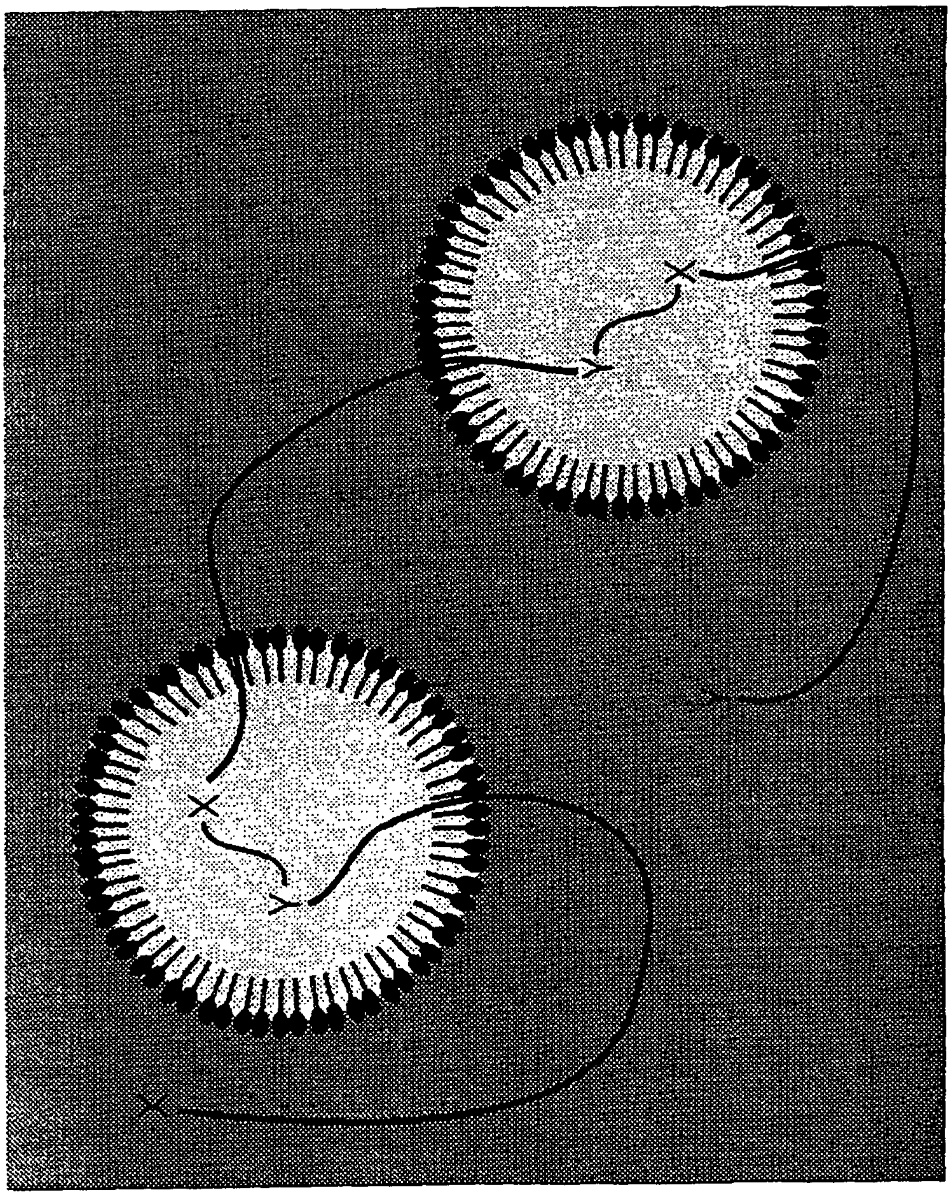

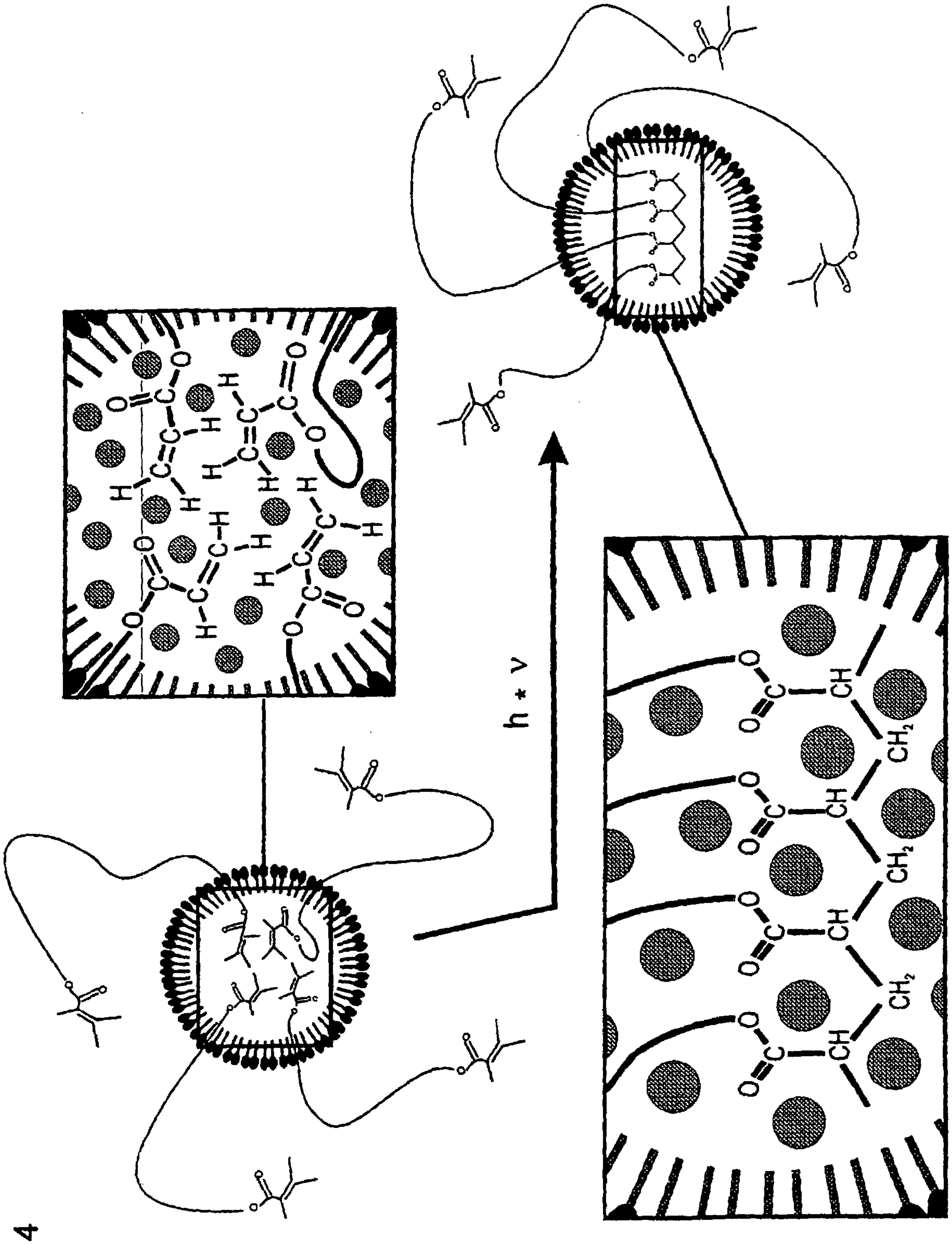


Fig. 4

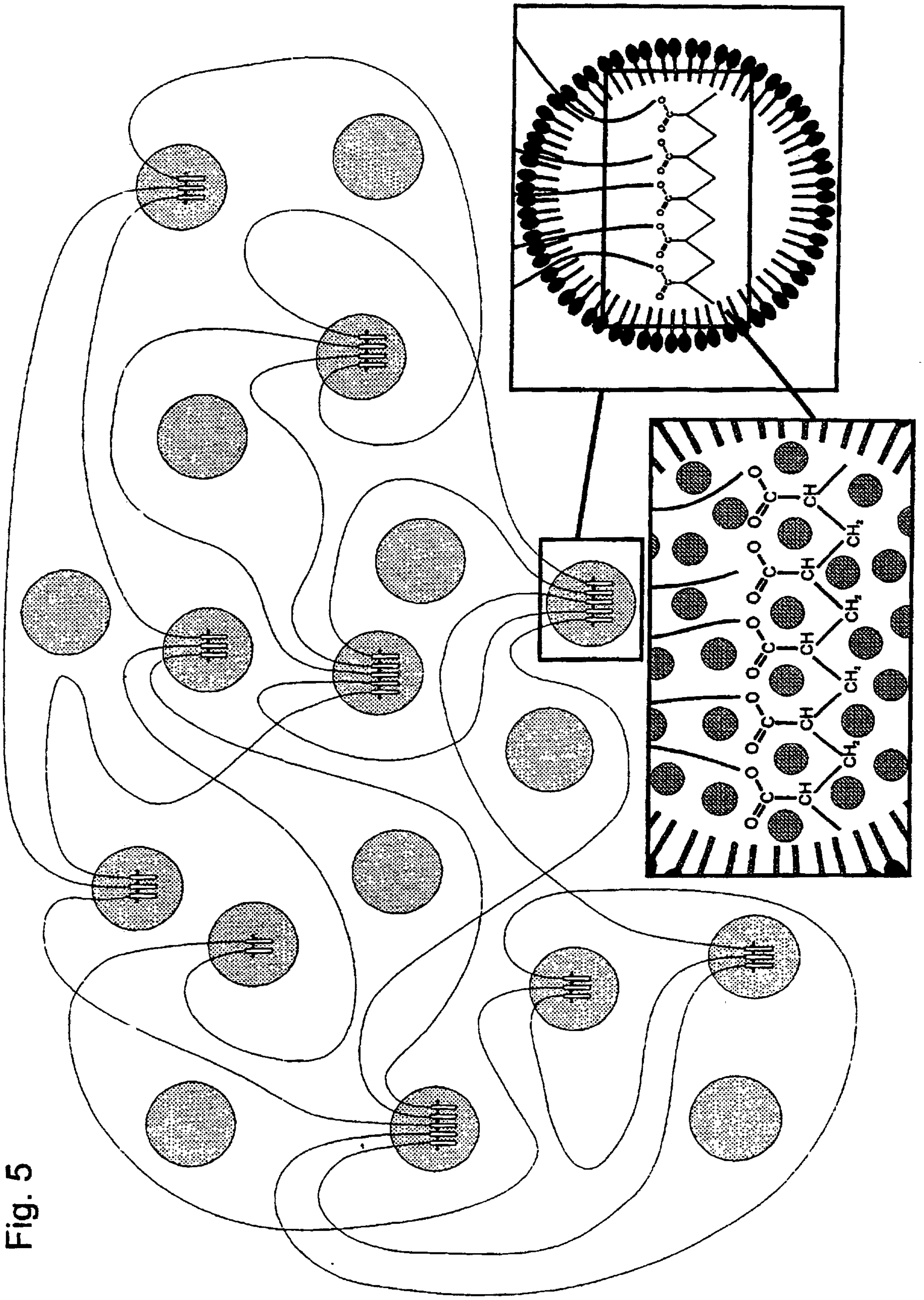

Fig. 5